# EUROPEAN PATENT APPLICATION

(11) **EP 4 461 313 A1**
(43) Date of publication of application: **13.11.2024**
(21) Application number: 23737284.2
(22) Date of filing: 04.01.2023
(51) Int. Cl.: A61K 45/00, A61K 39/395, A61K 47/68, A61P 35/00, A61P 35/02, A61P 35/04, C07K 14/47, C07K 16/28, C07K 16/40, C12Q 1/02

(54) **PHARMACEUTICAL COMPOSITION FOR TREATING CANCER**

(30) Priority: 04.01.2022 JP 2022000160
(71) Applicant: Fujiwara, Hiroshi, Kyoto-shi, Kyoto 603-8158 (JP)
(72) Inventor: Fujiwara, Hiroshi, Kyoto-shi, Kyoto 603-8158 (JP)
(74) Representative: Cabinet Becker et Associés
(86) International application number: PCT/JP2023/000019
(87) International publication number: WO 2023/132333

(57) **Abstract**

A pharmaceutical composition for treatment of cancer, comprising a substance binding to laeverin; a method for assessing cancer recurrence and/or metastasis; a method for determining a substance impairing a cancer cell expressing laeverin; a method for determining a factor influencing cancer recurrence and/or metastasis; and a use of laeverin as a biomarker for a cancer stem cell.

## Description

### TECHNICAL FIELD

The present disclosure relates to the field of pharmaceuticals. More specifically, the present disclosure relates to a pharmaceutical composition for treatment of cancer. The present disclosure relates to a method for assessing cancer recurrence, metastasis, or resistance. The present disclosure relates to a method for determining a substance impairing a cancer cell expressing laeverin. The present disclosure relates to a method for determining a factor influencing cancer recurrence, metastasis, or resistance. The present disclosure relates to a use of laeverin as a biomarker for a cancer stem cell.

### BACKGROUND ART

Early detection of cancer and appropriate treatment improves the survival rate of cancer patients. Some cancer cells can leave tumor tissue, enter blood vessels, and metastasize to other tissues. Circulating tumor cells (CTCs) in the blood are considered a factor in cancer metastasis and recurrence.

Laeverin is a unique glycoprotein expressed in placental extravillous trophoblast (EVT) cells (Patent Document 1 and Non-Patent Documents 1-6).

Many inoperable cancers are treated with chemotherapy and radiation therapy. However, it is challenging to eliminate cancer cells with these therapies completely. Cancer cells that are resistant to these therapies can contribute to cancer recurrence and/or metastasis. Cancer stem cells are of interest as cancer cells involved in cancer recurrence and/or metastasis. Cancer stem cells may exist within tumors and in a resting phase (G0 phase), a cellular state outside the replicative cell cycle. The cancer stem cells are capable of self-propagation and cell division into the same cancer stem cell and a differentiated cancer cell. The cell division is called asymmetric cell division. Because of these characteristics, cancer stem cells can resist conventional cancer therapies such as chemotherapy.

### CITATION LIST

Patent Document 1: JP 2005-160473 A
Non-Patent Document 1: Fujiwara H, et. al., "Promoting Roles of Embryonic Signals in Embryo Implantation and Placentation in Cooperation with Endocrine and Immune Systems. Int J Mol Sci. 2020 Mar 10;21(5):1885. doi: 10.3390/ijms21051885.
Non-Patent Document 2: Horie A, et. al., Laeverin/aminopeptidase Q induces trophoblast invasion during human early placentation. Hum Reprod. 2012 May;27(5):1267-76. doi: 10.1093/humrep/des068.
Non-Patent Document 3: Fujiwara H, et. al., Human extravillous trophoblasts express laeverin, a novel protein that belongs to membrane-bound gluzincin metallopeptidases. Biochem Biophys Res Commun. 2004 Jan 23;313(4):962-8. Non-Patent Document 4: Maruyama M, et. al., Histidine 379 of human laeverin/aminopeptidase Q, a nonconserved residue within the exopeptidase motif, defines its distinctive enzymatic properties. J Biol Chem. 2009 Dec 11;284(50):34692-702. doi: 10.1074/jbc.M109.066712.
Non-Patent Document 5: Maruyama M, et. al., Laeverin/aminopeptidase Q, a novel bestatin-sensitive leucine aminopeptidase belonging to the M1 family of aminopeptidases. J Biol Chem. 2007 Jul 13;282(28):20088-96. doi: 10.1074/jbc.M702650200.
Non-Patent Document 6: Imakawa K, et. al., Interferon-like sequence of ovine trophoblast protein secreted by embryonic trophectoderm. Nature. 1987 Nov 26-Dec 2;330(6146):377-9. doi: 10.1038/330377a0.

### SUMMARY OF THE INVENTION

### PROBLEMS TO BE SOLVED BY THE INVENTION

One object of the present disclosure is to provide a novel pharmaceutical composition for treatment of cancer. One object of the present disclosure is to provide a novel method for assessing cancer recurrence, metastasis, or resistance. One object of the present disclosure is to provide a novel method for determining a substance impairing a cancer cell expressing laeverin. One object of the present disclosure is to provide a novel method for determining a factor influencing cancer recurrence, metastasis, or resistance. One object of the present disclosure is to provide a novel biomarker for a cancer stem cell.

### SOLUTION TO THE PROBLEM

The inventor focused on genes whose expression levels change in cancer cells capable of propagating in a scaffold-independent manner to investigate a gene that plays an important role in circulating tumor cells (CTCs) in the blood. The inventor found that laeverin is not expressed in cancer cells cultured in a scaffold-dependent manner by adhesion culture but becomes expressed in cancer cells cultured in a scaffold-independent manner by suspension culture and is expressed in CTCs derived from biological samples. The inventor found that cancer cells expressing laeverin are involved in lymph node metastasis. The inventor further found that cancer cells expressing laeverin express indoleamine 2,3-dioxygenase-1 (IDO1), which is involved in suppressing T cell function and inducing regulatory T cell differentiation. The inventor also found that monocytes were induced to differentiate into IDO1-expressing dendritic cells upon contact with cells expressing laeverin. These findings indicate that cancer cells expressing laeverin provide a tumor microenvironment (TME) to evade an immune system attack. Based on these findings, the inventor has found that cancer cells expressing laeverin play an important role in cancer recurrence, metastasis or resistance.

The inventor further found that cancer cells surviving inside primary tumor tissue, surrounded by necrotic cancer cells, express laeverin. The inventor thought that the cancer cells expressing laeverin could survive in an environment where surrounding cancer cells would die, such as by suppressing their cell proliferation rate. The characteristic is similar to that of cancer stem cells. As described above, the cancer cells expressing laeverin can metastasize in lymph vessels, lymph nodes, and blood vessels, evading attacks from the immune system. Based on these findings, the inventor found that the cancer cells expressing laeverin must be cancer stem cells.

The present disclosure provides the following inventions.
[Item 1] A pharmaceutical composition for treatment of cancer, comprising a substance binding to laeverin.
[Item 2] The pharmaceutical composition according to item 1, wherein the substance binding to laeverin comprises an anti-laeverin antibody.
[Item 3] The pharmaceutical composition according to item 2, wherein the anti-laeverin antibody comprises
   (A) a heavy chain variable region comprising a CDR-H1 comprising an amino acid sequence: GYSFTDYI (SEQ ID NO. 1); a CDR-H2 comprising an amino acid sequence: INPYHAGI (SEQ ID NO. 2); and a CDR-H3 comprising an amino acid sequence: ARGSNYVYYYAMD (SEQ ID NO. 3), and/or a light chain variable region comprising a CDR-L1 comprising an amino acid sequence: SSVSY (SEQ ID NO. 4); a CDR-L2 comprising an amino acid sequence: ATS; and a CDR-L3 comprising an amino acid sequence: QQWSSNPPT (SEQ ID NO. 5),
   (B) a heavy chain variable region comprising a CDR-H1 comprising an amino acid sequence: GYTFTSYW (SEQ ID NO. 11); a CDR-H2 comprising an amino acid sequence: IDPYDSET (SEQ ID NO. 12); and a CDR-H3 comprising an amino acid sequence: ARDYGSRYYAMD (SEQ ID NO. 13), and/or a light chain variable region comprising a CDR-L1 comprising an amino acid sequence: ENVVTY (SEQ ID NO. 14); a CDR-L2 comprising an amino acid sequence: GAS; and a CDR-L3 comprising an amino acid sequence: GQGYSYP (SEQ ID NO. 15),
   (C) a heavy chain variable region comprising a CDR-H1 comprising an amino acid sequence: GYTFTSYW (SEQ ID NO. 11); a CDR-H2 comprising an amino acid sequence: IDPYDSET (SEQ ID NO. 12); and a CDR-H3 comprising an amino acid sequence: ARDYGSRYYAMD (SEQ ID NO. 13), and/or a light chain variable region comprising a CDR-L1 comprising an amino acid sequence: STISY (SEQ ID NO. 16); a CDR-L2 comprising an amino acid sequence: DTS; and a CDR-L3 comprising an amino acid sequence: QQWSSNPP (SEQ ID NO. 17), or
   (D) a heavy chain variable region comprising a CDR-H1 comprising an amino acid sequence: GYTFTDYY (SEQ ID NO. 18); and a CDR-H2 comprising an amino acid sequence: IYPRSGHS (SEQ ID NO. 19), and/or a light chain variable region comprising a CDR-L1 comprising an amino acid sequence: QSLLYSNIQKNY (SEQ ID NO. 20); a CDR-L2 comprising an amino acid sequence: WAS; and a CDR-L3 comprising an amino acid sequence: QQYYSYP (SEQ ID NO. 21).
[Item 4] The pharmaceutical composition according to item 2 or 3, wherein the anti-laeverin antibody possesses an ADCC activity or a CDC activity.
[Item 5] The pharmaceutical composition according to any one of items 1 to 4, wherein the substance binding to laeverin comprises a cytotoxic agent.
[Item 6] The pharmaceutical composition according to item 5, wherein the cytotoxic agent comprises at least one selected from the group consisting of a radioisotope, a chemotherapeutic agent, a toxin, and an enzyme.
[Item 7-1] The pharmaceutical composition according to any one of items 1 to 6, wherein the cancer is recurrent cancer, metastatic cancer or resistant cancer, or choriocarcinoma, placental trophoblast tumor, ovarian cancer, fallopian tube cancer, uterine cancer, cervical cancer, breast cancer, mammary gland cancer, glioblastoma, colon cancer, prostate cancer, or leukemia.
[Item 7-2] The pharmaceutical composition according to any one of items 1 to 6 for preventing recurrence, metastasis, or resistance of cancer.
[Item 8] A method for assessing cancer recurrence, metastasis, or resistance, comprising:
   detecting a cancer cell expressing laeverin in a biological sample derived from a subject in need thereof by using a substance binding to laeverin; and assessing recurrence, metastasis, or resistance of cancer, based on the detection result.
[Item 9] A method for determining a substance impairing a cancer cell expressing laeverin, comprising:
   culturing a cancer cell expressing laeverin under a condition that allows contact with a test substance, and determining the test substance as a substance impairing a cancer cell expressing laeverin based on the culture result.
[Item 10] A method for determining a factor influencing cancer recurrence, metastasis, or resistance, comprising:
   comparing a cancer cell expressing laeverin with a cancer cell that do not express laeverin but has the same origin as the cancer cell expressing laeverin to determine a difference in genetic or proteinaceous factor.
[Item 11] A use of laeverin as a biomarker for a cancer stem cell.

### BRIEF DESCRIPTION OF THE DRAWINGS

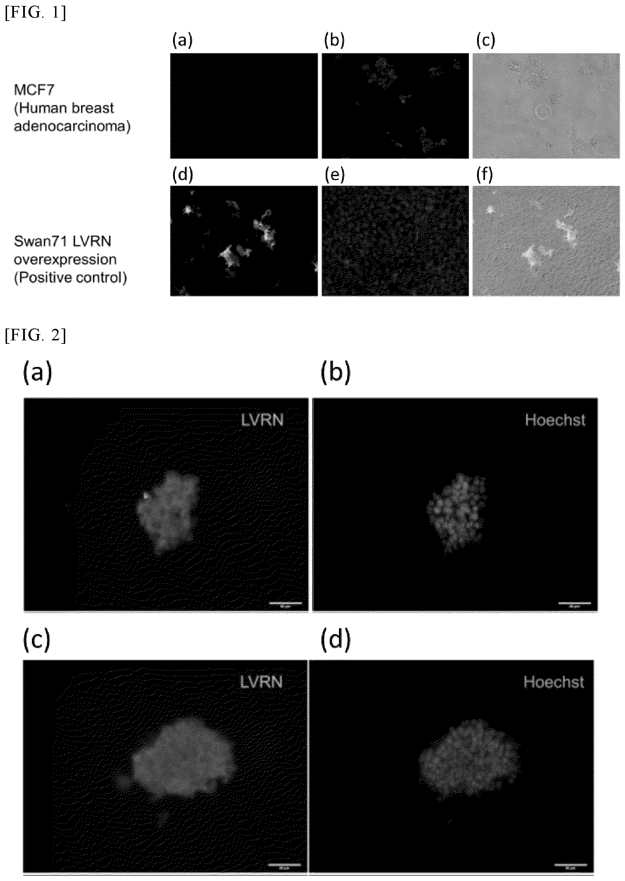

[FIG. 1] Figures 1a to 1c are images of adherent-cultured MCF7 cells: (FIG. 1a) laeverin (LVRN)-stained image, (FIG. 1b) nuclear-stained image, and (FIG. 1c) overlay of phase contrast image and the laeverin-stained image. Figures 1d to 1f are images of adherent-cultured Swan71 cells overexpressing: (FIG. 1d) laeverin-stained image, (FIG. 1e) nuclear-stained image, and (FIG. 1f) overlay of phase contrast image and the laeverin-stained image.
[FIG. 2] Figures. 2a and 2b are an image of laeverin stained with a monoclonal anti-laeverin antibody, 5-23 antibody and a nuclear-stained image of cell-suspension cultured MCF7 cell spheroids, respectively. Figures 2c and 2d are an image of laeverin stained with an anti-LVRN antibody and a nuclear-stained image of cell-suspension cultured MCF7 cell spheroids, respectively. The bars shown in the lower right of Figures 2a to 2d are scale bars indicating 50 µm.

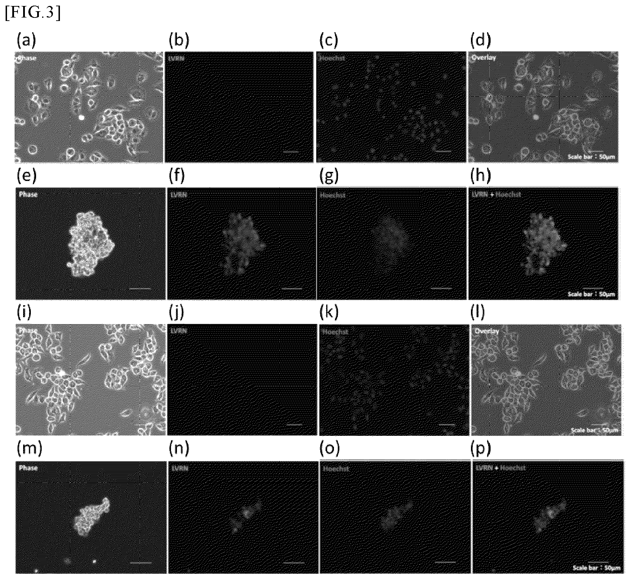

[FIG. 3] Figures 3a through 3d are images of adherent-cultured A375 cells: (FIG. 3a) phase contrast image, (FIG. 3b) laeverin-stained image, (FIG. 3c) nuclear-stained image, and (FIG. 3d) overlay of these images. Figures 3e to 3h are images of cell-suspension cultured A375 cell spheroids: (FIG. 3e) phase contrast image, (FIG. 3f) laeverin-stained image, (FIG. 3g) nuclear-stained image, and (FIG. 3h) overlay of the laeverin-stained image and nuclear-stained image. Figures 3i to 3l are images of adherent-cultured A2780 cells: (FIG. 3i) phase contrast image, (FIG. 3j) laeverin-stained image, (FIG. 3k) nuclear-stained image, and (FIG. 3l) overlay of these images. Figures 3m to 3p are images of cell-suspension cultured A2780 cell spheroids: (FIG. 3m) phase contrast image, (FIG. 3n) laeverin-stained image, (FIG. 3o) nuclear-stained image, and (FIG. 3p) overlay of the laeverin-stained and nuclear-stained images. The bars shown in the lower right of Figures 3a to 3p are scale bars indicating 50 µm.

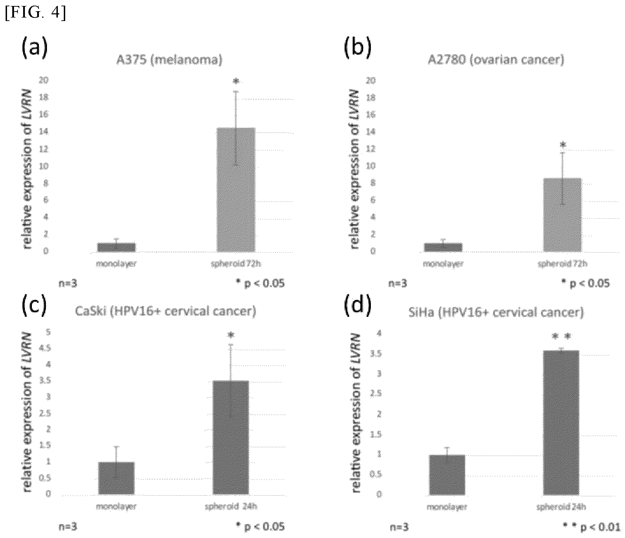

[FIG. 4] Figure 4a is a bar graph showing a relative amount of mRNA transcripts of laeverin in cell-suspension cultured A375 cell line (spheroids) to the normalized amount of the mRNA transcripts (normalized to 1) in adherent-cultured A375 cell line (monolayer). Figure 4b is a bar graph showing a relative amount of the laeverin mRNA transcripts in A2780 cell line. Figure 4c is a bar graph showing a relative amount of the laeverin mRNA transcripts in CaSki cell line. Figure 4d is a bar graph showing a relative amount of the laeverin mRNA transcripts in SiHa cell line.

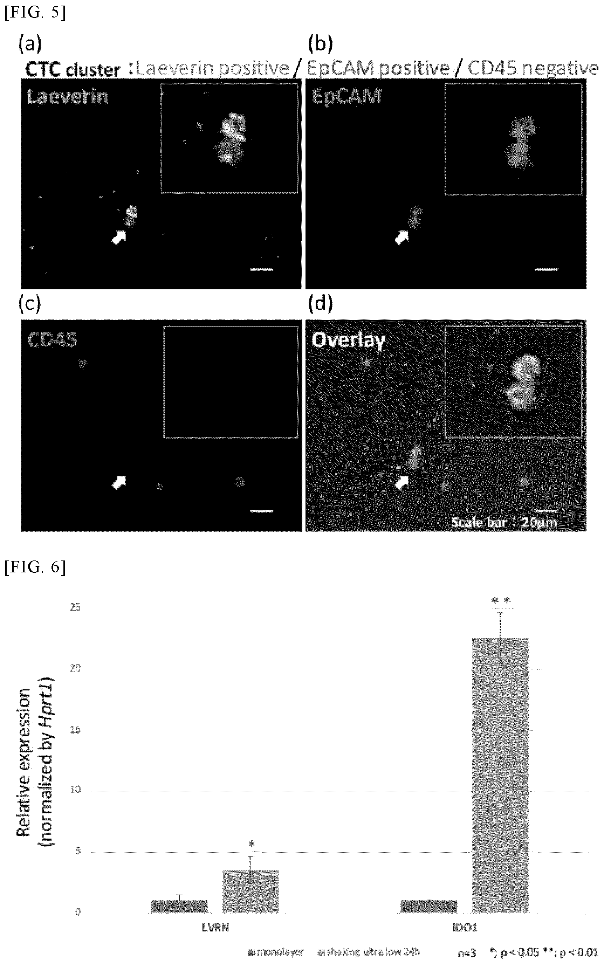

[FIG. 5] Figure 5a is a fluorescent image showing a laeverin-positive spheroid (white arrow). Figure 5b is a fluorescent image showing that the spheroid in Figure 5a (white arrow) is EpCAM positive. Figure 5c is a fluorescent image showing that the spheroid in Figure 5a (white arrow) is CD45 negative. Figure 5d is an overlay image of the fluorescent images of Figures 5a-5c. The images circled by white squares in Figures 5a to 5d are magnified images of the spheroids indicated by the white arrows. The bars shown in the lower right of Figures 5a to 5d are scale bars indicating 20 µm.
[FIG. 6] Figure 6 is a bar graph showing relative transcription levels of laeverin (LVRN) and IDO1 in spheroids of cancer cells, respectively.

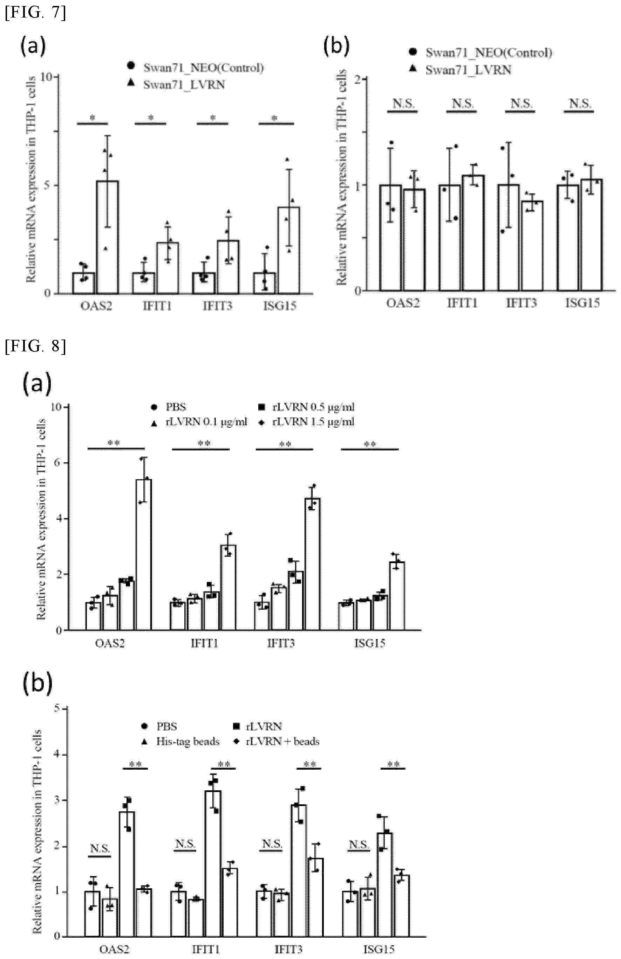

[FIG. 7] Figure 7a is a bar graph showing relative expression levels of four genes (OAS2, IFIT1, IFIT3, and ISG15) in THP-1 cells cultured under a condition that allows direct contact with Swan71 cells. Figure 7b is a bar graph showing the relative expression levels of the genes in THP-1 cells cultured under a condition that does not allow direct contact with Swan71 cells.
[FIG. 8] Figure 8a is a bar graph showing the relative expression levels of the four genes (OAS2, IFIT1, IFIT3, and ISG15) in THP-1 cells cultured in the presence of various concentrations of recombinant laeverin (rLVRN). Figure 8b is a bar graph showing the relative expression levels of the genes in THP-1 cells cultured in the presence of rLVRN-immobilized beads (◆: rLVRN+beads) or free-form of rLVRN (■: rLVRN).

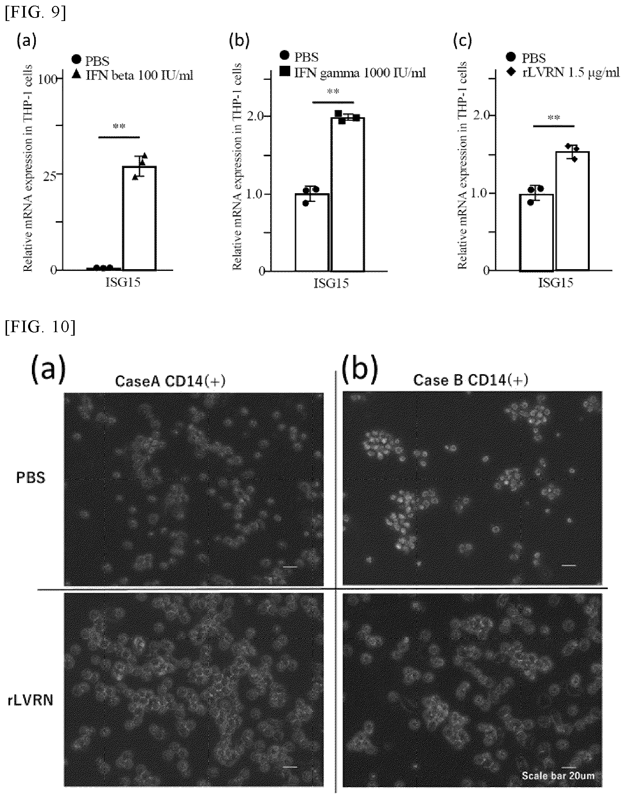

[FIG. 9] Figures 9a to 9c are bar graphs showing relative expression levels of ISG15 in THP-1 cells cultured in the presence of INF-β (FIG. 9a), INF-γ (FIG. 9b), and rLVRN (FIG. 9c), respectively.
[FIG. 10] The upper and lower figures of FIG. 10a are micrographic images of CD14-positive cells derived from peripheral blood mononuclear cells (PBMCs) from subject A, cultured in a medium supplemented with PBS and rLVRN, respectively. The upper and lower figures of FIG. 10b are micrographic images of CD14-positive cells derived from PBMCs from subject B cultured in a medium supplemented with PBS and rLVRN, respectively.

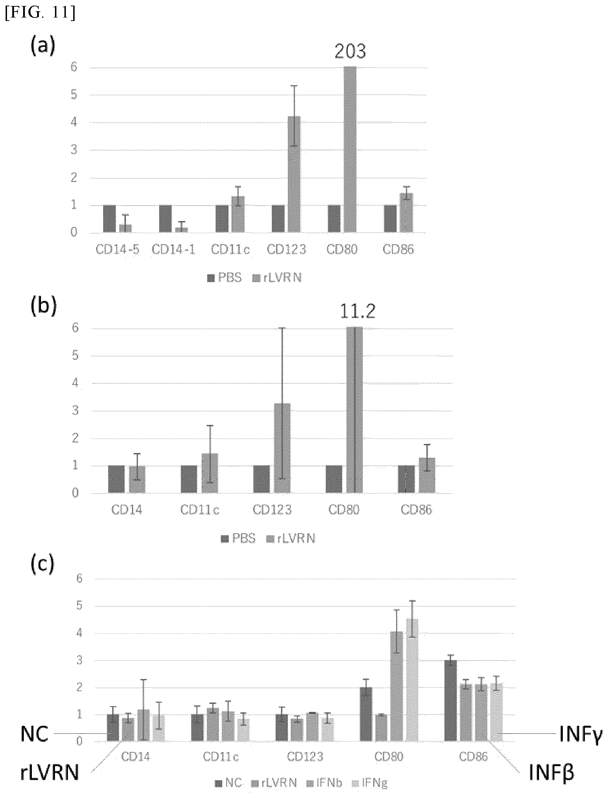

(FIG. 11] Figures 11a to 11c are bar graphs showing gene expressions in CD14-positive cells (FIG. 11a), PBMCs (FIG. 11b), and THP-1 (FIG. 11c) cultured in the presence of rLVRN.

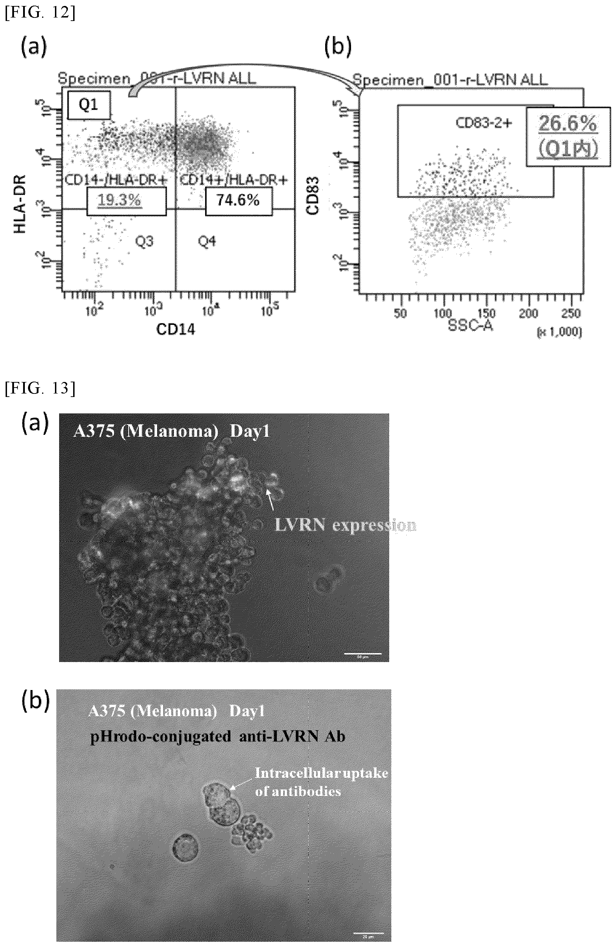

[FIG. 12] Figure 12a is a scattergram of CD14 and HLA-DR in PBMCs cultured in the presence of rLVRN. Figure 12b is a scattergram of CD83 for the Q1 fraction of Figure 12a.
[FIG. 13] Figure 13a is a stained image showing the expression of laeverin in A375 melanoma cells cultured by a Hanging Drop method (1,000 cells/medium drop of 20 µL). Figure 13b is a stained image showing intracellular uptake of anti-LVRN antibodies by A375 melanoma cells cultured by the Hanging Drop method (10 cells/20 µL of medium drop) in the presence of pHrodo-conjugated anti-LVRN antibody.

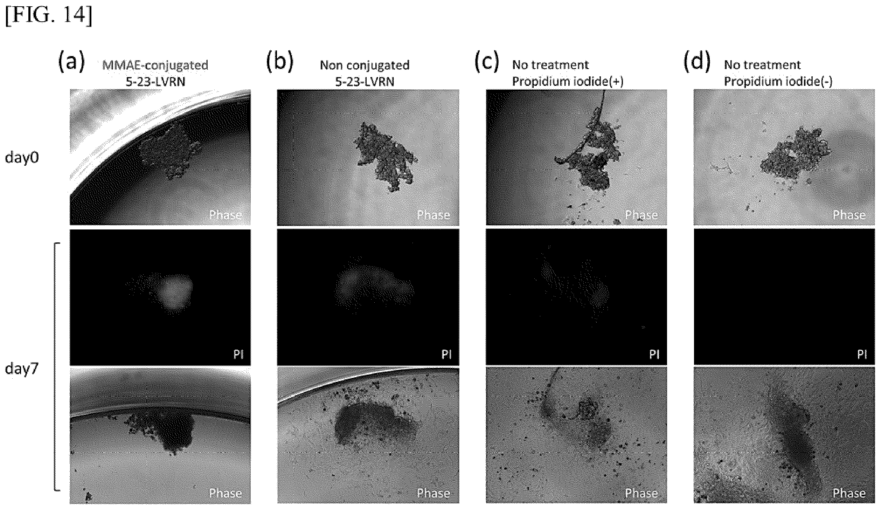

[FIG. 14] Figure 14a is a Propidium Iodide (PI) stained image of a spheroid of SKOV3 ovarian cancer cells cultured in the presence of 5-23 antibody conjugated with monomethyl auristatin E (MMAE). Figure 14b is a PI-stained image of an SKOV3 spheroid cultured in the presence of 5-23 antibody. Figure 14c is a PI-stained image of an SKOV3 spheroid. Figure 14d is an image of an SKOV3 spheroid without PI staining.

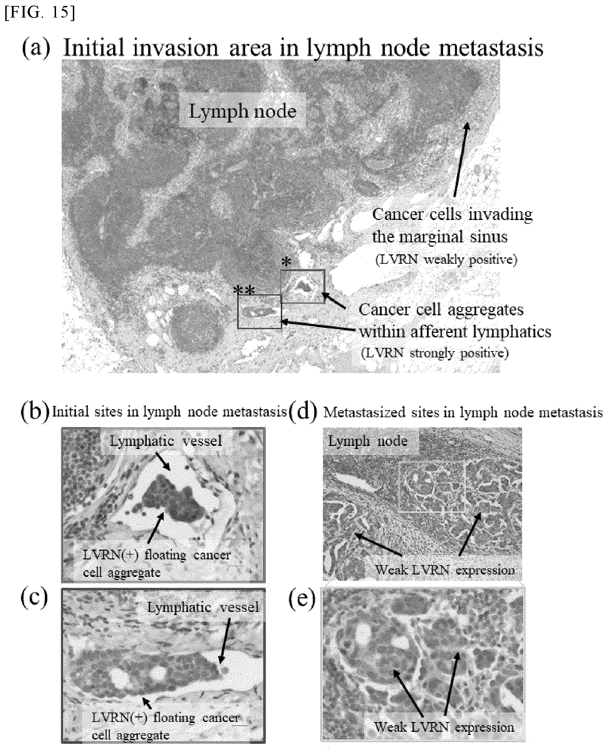

[FIG. 15] Figure 15a is a stained image of an initial invasion area of lymph node metastasis. Figure 15b is an enlarged image of the site surrounded by a solid line with an asterisk * in FIG. 15a. Figure 15c is an enlarged image of the site surrounded by a solid line with double asterisks ** in FIG. 15a. Figure 15d is a stained image of a site where lymph node metastasis was established. Figure 15e is an enlarged image of the site surrounded by a solid line in Figure 15d.

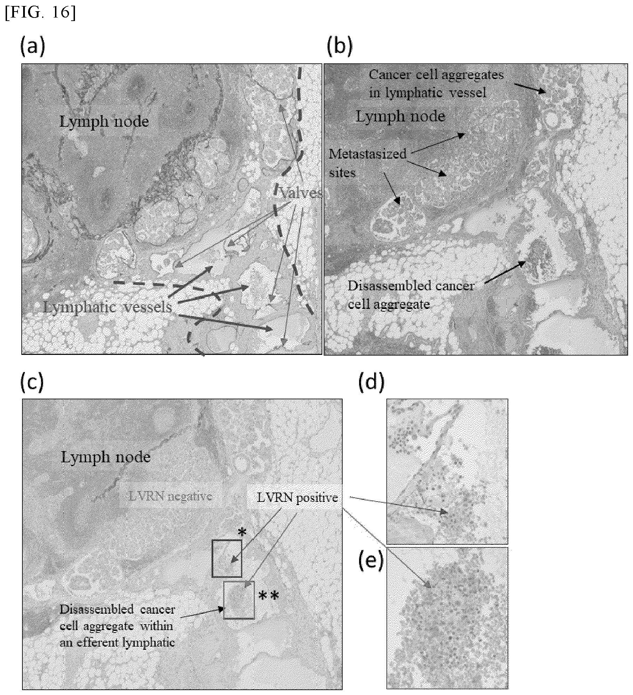

[FIG. 16] Figure 16a is a stained image of lymphatic endothelium around an efferent lymphatic in a lymph node. The lymphatic endothelium was stained with Podoplanin. Figure 16b is a HE-stained image of the lymph node near the efferent lymphatic. Figure 16c is a stained image of the lymph node near the efferent lymphatic. Figure 16d is an enlarged image of the site surrounded by a solid line with an asterisk * in FIG. 16c. Figure 16e is an enlarged image of the site surrounded by a solid line with double asterisks ** in FIG. 16c.

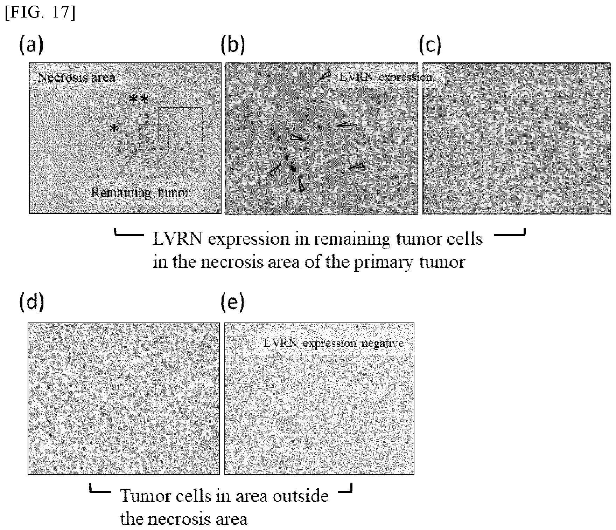

[FIG. 17] Figure 17a is a stained image of a necrosis area within the primary tumor. Figure 17b is an enlarged image of the area surrounded by a solid line with an asterisk * in FIG. 17a. Figure 17b is an enlarged image of the area surrounded by a solid line with double asterisks ** in FIG. 17a. Figure 17d is a HE-stained image of an area other than necrosis areas within the primary tumor. Figure 17e is a stained image of the area other than the necrosis areas within the primary tumor.

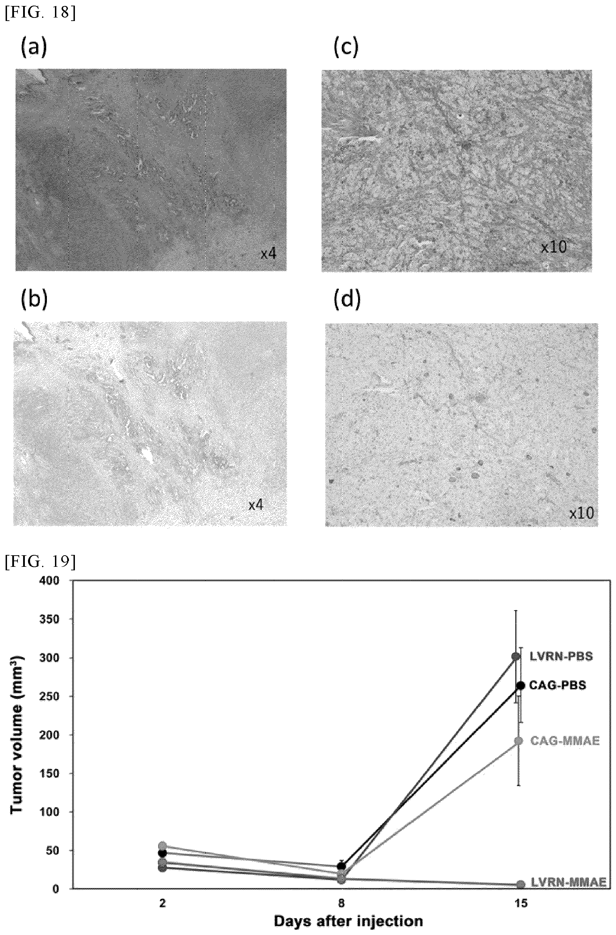

[FIG. 18] Figure 18a is a HE-stained image of a section of ovarian cancer after the application of chemotherapy (TC therapy). Figure 18b is an image of the ovarian cancer section after the chemotherapy application, immunologically stained with an anti-laeverin antibody. Figure 18c is a HE-stained image of a section of ovarian cancer after the application of chemotherapy (DC therapy). Figure 18d is an image of the ovarian cancer section after the chemotherapy application, immunologically stained with an anti-laeverin antibody.
[FIG. 19] Figure 19 is a line graph showing tumor sizes in mice into which cancer cells were transplanted.

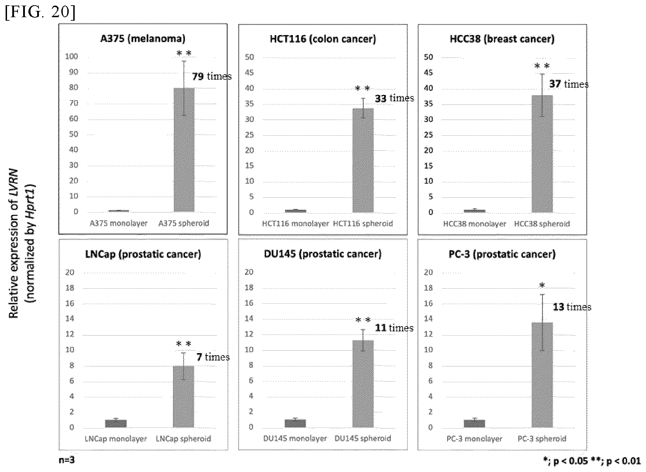

[FIG. 20] Figure 20 is a series of bar graphs showing the expression levels of laeverin in spheroids formed of cancer cell lines.

### DESCRIPTION OF EMBODIMENTS

### (Pharmaceutical composition)

One aspect of the present disclosure relates to a pharmaceutical composition for treatment of cancer, comprising a substance binding to laeverin.

The term "laeverin" or "LVRN" refers to a glycoprotein determined as a cell surface antigen of placental extravillous trophoblast (EVT) cells. The amino acid sequence of laeverin is publicly available at databank websites. Laeverin may be, for example, a protein that has 80%, 85%, 90%, 95%, 96%, 97%, 98%, or 99% or more sequence identity to the amino acid sequence shown in SEQ ID No. 10 when calculated with a homology search software with default parameters (e.g. BLAST and FASTA). Laeverin comprises a mutant having a natural mutation and keeping the original function. The mutation may be a deletion, substitution, or addition in a given amino acid sequence or a combination thereof.

For example, human laeverin consists essentially of 990 amino acids (SEQ ID NO. 10). Laeverin, consisting essentially of the amino acid sequence shown in SEQ ID NO. 10, may include, for example, a post-translational modification such as glycosylation. Laeverin may comprise an amino acid sequence, for example, including mutations in the amino acid sequence shown in SEQ ID NO. 10 and having at least 85% (preferably no less than 90%, more preferably no less than 95%, no less than 97%, no less than 98%, or no less than 99%) sequence identity with the amino acid sequence shown in SEQ ID NO. 10, as long as it has the original function.

The term "sequence identity" refers to a percentage of amino acids or nucleotides that match between two amino acid or polynucleotide sequences that are optimally aligned. Sequence identity can be calculated with commercially or publicly available software such as BLAST+. The "deletion" of an amino acid means the loss of an amino acid residue at any position in a given amino acid sequence. The "addition" of an amino acid means the increase or insertion of an amino acid residue at any position in a given amino acid sequence. The "substitution" of an amino acid means the replacement of an amino acid residue at any position in a given amino acid sequence with a different amino acid residue. The substitution of amino acid may be, for example, conservative substitution.

A "substance binding to laeverin" comprises any substance binding to laeverin with a prescribed binding ability. For example, a substance binding to laeverin binds to laeverin consisting of the amino acid sequence shown in SEQ ID No. 10 (MGPPSSSGFYVSHAVALLLAGLVAALLLALAVLAALYGHCERVPPSELPG LRDSEAESSPPLRQKPTPTPKPSSARELAVTTTPSNWRPPGPWDQLRLPPW LVPLHYDLELWPQLRPDELPAGSLPFTGRVNITVRCTVATSRLLLHSLFQD CERAEVRGPLSPGTGNATVGRVPVDDVWFALDTEYMVLELSEPLKPGSSY ELQLSFSGLVKEDLREGLFLNVYTDQGERRALLASQLEPTFARYVFPCFDE PALKATFNITMIHHPSYVALSNMPKLGQSEKEDVNGSKWTVTTFSTTPHM PTYLVAFVICDYDHVNRTERGKEIRIWARKDAIANGSADFALNITGPIFSFL EDLFNISYSLPKTDIIALSSFDNHAMENWGLMIFDESGLLLEPKDQLTEKKT LISYVVSHEIGHQWFGNLVTMNWWNNIWLNEGFASYFEFEVINYFNPKLP RNEIFFSNILHNILREDHALVTRAVAMKVENFKTSEIQELFDIFTYSKGASM ARMLSCFLNEHLFVSALKSYLKTFSYSNAEQDDLWRHFQMAIDDQSTVIL PATIKNIMDSWTHQSGFPVITLNVSTGVMKQEPFYLENIKNRTLLTSNDTW IVPILWIKNGTTQPLVWLDQSSKVFPEMQVSDSDHDWVILNLNMTGYYRV NYDKLGWKKLNQQLEKDPKAIPVIHRLQLIDDAFSLSKNNYIEIETALELT KYLAEEDEIIVWHTVLVNLVTRDLVSEVNIYDIYSLLKRYLLKRLNLIWNI YSTIIRENVLALQDDYLALISLEKLFVTACWLGLEDCLQLSKELFAKWVDH PENEIPYPIKDVVLCYGIALGSDKEWDILLNTYTNTTNKEEKIQLAYAMSC SKDPWILNRYMEYAISTSPFTSNETNIIEVVASSEVGRYVAKDFLVNNWQA VSKRYGTQSLINLIYTIGRTVTTDLQIVELQQFFSNMLEEHQPIRVHANLQT IKNENLKNKKLSARIAAWLRRNT) with a dissociation constant of no more than 10⁻⁶ M, no more than 10⁻⁷ M, no more than 10⁻⁸ M, no more than 10⁻⁹ M, or no more than 10⁻¹⁰ M. A substance binding to laeverin binds to laeverin, for example, with a given binding capacity, while it does not substantially bind to substances other than laeverin. A substance that "does not substantially bind" to substances other than laeverin comprises a substance binding to substances other than laeverin with a dissociation constant of no less than 10⁻⁴ M. The substance that does not substantially bind to substances other than laeverin is, for example, a substance binding to substances other than laeverin with a dissociation constant of no less than 10⁻³ M or no less than 10⁻² M.

The substance binding to laeverin has a molecular weight of, for example, no less than 10 kDa, no less than 20 kDa, no less than 30 kDa, no less than 50 kDa, no less than 75 kDa, no less than 100 kDa, no less than 125 kDa, no less than 150 kDa, no less than 175 kDa, or no less than 200 kDa. The substance binding to laeverin has a molecular weight of, for example, less than 500 kDa, less than 450 kDa, less than 400 kDa, less than 350 kDa, less than 300 kDa, less than 250 kDa, or less than 200 kDa.

The substance binding to laeverin is, for example, a small molecule compound, protein (e.g., antibodies), DNA, RNA, small interfering RNA, or antisense oligonucleotide. The substance binding to laeverin is, for example, an anti-laeverin antibody or a receptor to which laeverin binds, or a fragment thereof. The substance binding to laeverin is, for example, a monoclonal anti-laeverin antibody or polyclonal anti-laeverin antibodies. The anti-laeverin antibody is, for example, a human or humanized antibody. The substance binding to laeverin is, for example, any antigen-binding moiety that competes with an anti-laeverin antibody or any fusion protein containing the antigen-binding moiety.

The term "antibody" refers to a substance capable of binding to a target via at least one antigen recognition site that exists in a variable region of an immunoglobulin molecule. Antibody is, for example, an intact polyclonal or monoclonal antibody, a chimeric antibody, or an antigen-binding portion thereof. Antigen-binding site is, for example, Fab, Fab', F(ab')₂, Fv, a fragment containing complementarity determining regions (CDRs), single chain antibody (scFv), a diabody, a triabody, or a tetrabody. Antibody is any class of antibody, such as IgG, IgA, or IgM, or an antigen-binding fragment thereof. The "variable region" of an antibody may be a variable region of antibody light chain (V_{L}) or a variable region of antibody heavy chain (V_{H}). The variable region of an antibody heavy chain or light chain includes three complementarity determining regions (CDRs) and four framework regions (FRs).

CDRs can be identified, for example, according to IgBLAST provided by the National Center for Biotechnology Information (NCBI). The heavy chain variable region of an antibody includes CDR-H1, CDR-H2, and CDR-H3. The light chain variable region of the antibody includes CDR-L1, CDR-L2, and CDR-L3.

The term "monoclonal antibody" refers to an antibody obtained from a population of substantially homologous antibodies. The substantially homologous antibodies do not exclude any antibody containing a natural mutation. A monoclonal antibody can be prepared according to known methods.

The term "humanized antibody" refers to an antibody originated from wholly or partially non-human, comprising non-human-originated heavy and light chains in which amino acids included in the framework regions are replaced to avoid or minimize immune responses in humans. A humanized antibody, for example, comprises or consists essentially of human C_{H} and C_{L} domains in its constant domain. A humanized antibody comprises, for example, a human-originated constant domain.

The term "human antibody" refers to an antibody comprising human-originated variable region consisting of regions of framework and CDRs and constant region. The term "chimeric antibody" refers to an antibody comprising a constant region originated from a specific species in which amino acids are replaced by those originated from another species or a portion thereof. Chimeric antibody is, for example, an antibody comprising a variable region originated from mouse and a constant region originated from human.

Antibody can be prepared, for example, by isolation from natural sources (e.g., vertebrates). Antibody can be prepared, for example, from mammals such as a human, a monkey, a pig, a horse, a rabbit, a dog, a cat, and a mouse; birds such as a chicken; fish such as a shark; or camelids such as a llama. Antibody can be artificially produced, for example, according to techniques such as genetic engineering techniques. Antibody can be prepared, for example, by selection from an antibody library based on its binding ability to laeverin. Antibody can be prepared, for example, according to phage display methods.

A cytotoxic agent-conjugated substance binding to laeverin is preferred because it can efficiently inhibit or arrest the growth of cancer cells expressing laeverin or kill or destroy the cancer cells. A pharmaceutical composition for treating cancer preferably comprises an anti-laeverin antibody with antibody-dependent cytotoxic activity (ACDD activity) or complement-dependent cytotoxic activity (CDC activity). A pharmaceutical composition for treating cancer preferably comprises a cytotoxic agent-conjugated substance binding to laeverin.

The term "antibody-dependent cytotoxic activity (ACDD activity)" refers to an activity to damage a target cell by an immune cell belonging to the immune system. The immune cell includes an Fc receptor capable of binding to an Fc region of antibody. The ACDD activity is caused by the immune cell (e.g., macrophage or natural killer cell), which kills a target cell (e.g., cancer cell expressing laeverin) to which antibodies including an Fc region bind. The term "complement-dependent cytotoxic activity (CDC activity)" refers to an activity to damage a target cell through the binding of a complement system protein. The complement system protein is capable of binding to an Fc region of antibody. The CDC activity is caused by the complement system protein, which lysis a target cell (e.g., cancer cells expressing laeverin) to which antibodies including Fc region bind. The term "Fc region" refers to a region corresponding to the fragment obtained by partial degradation of an intact antibody with papain, a proteolytic enzyme.

A substance binding to laeverin is, for example, an anti-laeverin antibody possessing ACDD or CDC activity. The anti-laeverin antibody includes, for example, an Fc region. The anti-laeverin antibody is, for example, a monoclonal or polyclonal antibody, preferably a monoclonal antibody. A cytotoxic agent-conjugated substance binding to laeverin includes, for example, an anti-laeverin antibody possessing ACDD or CDC activity and a cytotoxic agent described below. A cytotoxic agent-conjugated substance binding to laeverin includes, for example, an anti-laeverin antibody possessing ACDD or CDC activity and at least one selected from the group consisting of radioisotopes, chemotherapeutic agents, enzymes or fragments thereof, and toxins.

A substance binding to laeverin is, for example, an anti-laeverin antibody (e.g., mouse, humanized, or human antibody) comprising
a heavy chain variable region that includes CDR-H1 comprising or consisting of the amino acid sequence: GYSFTDYI (SEQ ID NO. 1); CDR-H2 comprising or consisting of the amino acid sequence: INPYHAGI (SEQ ID NO. 2); and CDR-H3 comprising or consisting of the amino acid sequence: ARGSNYVYYYAMD (SEQ ID NO. 3), and/or
a light chain variable region that includes CDR-L1 comprising or consisting of the amino acid sequence: SSVSY (SEQ ID NO. 4); CDR-L2 comprising or consisting of the amino acid sequence: ATS; and CDR-L3 comprising or consisting of QQWSSNPPT (SEQ ID NO. 5).

A substance binding to laeverin is, for example, an anti-laeverin antibody (e.g., antigen-binding portion, or Fab, Fab', F(ab')₂, or Fv) comprising a heavy chain variable region comprising or consisting essentially of an amino acid sequence: 
MSSPQPLKTLTLTMGGSWIFLFLLSGTAGAHSEIQLQQTGPELVKPGASVK
ISCKASGYSFTDYIMLWVKQSHGKSLEWIGNINPYHAGISYNLKFKGKATL
TVDTSSSTAYMQLNSLTSEDSAVYYCARGSNYVYYYAMDYWGQGTSVT
VSSAKTTPPSVYPLAPGSAAQTNSMVTLGCLVKGYFPEPVTVTWNSGSLSS
GVHTFPAVLQSDLYTLSSSVTVPSSTWPSETVTCNVAHPASSTKVDKKIVP
RDCGCKPCICTVPEVSSVFIFPPKPKDVLTITLTPKVTCVVVDISKDDPEVQ
FSWFVDDVEVHTAQTQPREEQFNSTFRSVSELPIMHQDWLNGKEFKCRVN
SAAFPAPIEKTISKTKGRPKAPQVYTIPPPKEQMAKDKVSLTCMITDFFPED
ITVEWQWNGQPAENYKNTQPIMDTDGSYFVYSKLNVQKSNWEAGNTFTC
SVLHEGLHNHHTEKSLSHSPGK (SEQ ID NO. 6);
 and/or a light chain variable region comprising or consisting essentially of amino acid sequence: MDFQVQIFSFLLISASVIMSRGQIVLSQSPAILSASPGEKVTMTCRASSSVSY MHWYQQKPGSSPKPWIFATSNLASGVPARFSGSGSGTSYSLTISRVEAEDA ATYYCQQWSSNPPTFGGGTKLEIKRADAAPTVSIFPPSSEQLTSGGASVVC FLNNFYPKDINVKWKIDGSERQNGVLNSWTDQDSKDSTYSMSSTLTLTKD EYERHNSYTCEATHKTSTSPIVKSFNRNEC (SEQ ID NO. 7). The amino acid sequence shown in SEQ ID NO. 6 (also referred to as 5-23 antibody HC) comprises the amino acid sequence shown in SEQ ID NO. 1, the amino acid sequence shown in SEQ ID NO. 2, and the amino acid sequence shown in SEQ ID NO. 3. The amino acid sequence shown in SEQ ID NO. 7 (also referred to as 5-23 antibody LC) comprises the amino acid sequence shown in SEQ ID NO. 4 and the amino acid sequence shown in SEQ ID NO. 5.

A substance binding to laeverin is, for example, an anti-laeverin antibody (e.g., mouse, humanized, or human antibody) comprising
a heavy chain variable region that includes CDR-H1 comprising or consisting of the amino acid sequence: GYTFTSYW (SEQ ID NO. 11); CDR-H2 comprising or consisting of the amino acid sequence: IDPYDSET (SEQ ID NO. 12); and CDR-H3 comprising or consisting of the amino acid sequence: ARDYGSRYYAMD (SEQ ID NO. 13), and/or
a light chain variable region that includes CDR-L1 comprising or consisting of the amino acid sequence: ENVVTY (SEQ ID NO. 14); CDR-L2 comprising or consisting of the amino acid sequence: GAS; and CDR-L3 comprising or consisting of GQGYSYP (SEQ ID NO. 15).

A substance binding to laeverin is, for example, an anti-laeverin antibody (e.g., mouse, humanized, or human antibody) comprising
a heavy chain variable region that includes CDR-H1 comprising or consisting of the amino acid sequence: GYTFTSYW (SEQ ID NO. 11); CDR-H2 comprising or consisting of the amino acid sequence: IDPYDSET (SEQ ID NO. 12); and CDR-H3 comprising or consisting of the amino acid sequence: ARDYGSRYYAMD (SEQ ID NO. 13), and/or
a light chain variable region that includes CDR-L1 comprising or consisting of the amino acid sequence: STISY (SEQ ID NO. 16); CDR-L2 comprising or consisting of the amino acid sequence: DTS; and CDR-L3 comprising or consisting of QQWSSNPP (SEQ ID NO. 17).

A substance binding to laeverin is, for example, an anti-laeverin antibody (e.g., mouse, humanized, or human antibody) comprising
a heavy chain variable region that includes CDR-H1 comprising or consisting of the amino acid sequence: GYTFTDYY (SEQ ID NO. 18); and CDR-H2 comprising or consisting of the amino acid sequence: IYPRSGHS (SEQ ID NO. 19), and/or
a light chain variable region that includes CDR-L1 comprising or consisting of the amino acid sequence: QSLLYSNIQKNY (SEQ ID NO. 20); CDR-L2 comprising or consisting of the amino acid sequence: WAS; and CDR-L3 comprising or consisting of QQYYSYP (SEQ ID NO. 21).

A substance binding to laeverin is, for example, an anti-laeverin antibody (e.g., antigen-binding portion, or Fab, Fab', F(ab')₂, or Fv) comprising a heavy chain variable region comprising or consisting essentially of an amino acid sequence: 
MGWSYIILFLLATATCVHSQVQLQQPGAELVRPGASVKLSCKASGYTFTS
YWMNWVRQRPEQGLEWIGRIDPYDSETHYNQKFKDKAILTVDKSSSTVH
MQLSSLTSEDSAVYHCARDYGSRYYAMDYWGQGTSVTVSSAKTTPPSVY
PLAPGSAAQTNSMVTLGCLVKGYFPEPVTVTWNSGSLSSGVHTFPAVLQS
DLYTLSSSVTVPSSTWPSETVTCNVAHPASSTKVDKKIEPRDCGCKPCICT
VPEVSSVFIFPPKPKDVLTITLTPKVTCVVVDISKDDPEVQFSWFVDDVEVH
TAQTQPREEQFNSTFRSVSELPIMHQDWLNGKEFKCRVNSAAFPAPIEKTIS
KTKGRPKAPQVYTIPPPKEQMAKDKVSLTCMITDFFPEDITVEWQWNGQP
AENYKNTQPIMDTDGSYFVYSKLNVQKSNWEAGNTFTCSVLHEGLHNHH
TEKSLSHSPGK (SEQ ID NO. 22);
 and/or a light chain variable region comprising or consisting essentially of amino acid sequence: MGIKMESQTLVFISILLWLYGADGNIVMTQSPKSMSMSVGERVTLTCKAS ENVVTYVSWYQQKPEQSPKLLIYGASNRYTGVPDRFTGSGSATDFTLTISS VQAEDLADYHCGQGYSYPYTFGGGTKLEIKRADAAPTVSIFPPSSEQLTSG GASVVCFLNNFYPKDINVKWKIDGSERQNGVLNSWTDQDSKDSTYSMSS TLTLTKDEYERHNSYTCEATHKTSTSPIVKSFNRNEC (SEQ ID NO. 23). The amino acid sequence shown in SEQ ID NO. 22 (also referred to as 94-2/129-5 antibody HC) comprises the amino acid sequence shown in SEQ ID NO. 11, the amino acid sequence shown in SEQ ID NO. 12, and the amino acid sequence shown in SEQ ID NO. 13. The amino acid sequence shown in SEQ ID NO. 23 (also referred to as 94-2 antibody LC) comprises the amino acid sequence shown in SEQ ID NO. 14 and the amino acid sequence shown in SEQ ID NO. 15.

A substance binding to laeverin is, for example, an anti-laeverin antibody (e.g., antigen-binding portion, or Fab, Fab', F(ab')₂, or Fv) comprising a heavy chain variable region comprising or consisting essentially of an amino acid sequence: 
MGWSYIILFLLATATCVHSQVQLQQPGAELVRPGASVKLSCKASGYTFTS
YWMNWVRQRPEQGLEWIGRIDPYDSETHYNQKFKDKAILTVDKSSSTVH
MQLSSLTSEDSAVYHCARDYGSRYYAMDYWGQGTSVTVSSAKTTPPSVY
PLAPGSAAQTNSMVTLGCLVKGYFPEPVTVTWNSGSLSSGVHTFPAVLQS
DLYTLSSSVTVPSSTWPSETVTCNVAHPASSTKVDKKIEPRDCGCKPCICT
VPEVSSVFIFPPKPKDVLTITLTPKVTCVVVDISKDDPEVQFSWFVDDVEVH
TAQTQPREEQFNSTFRSVSELPIMHQDWLNGKEFKCRVNSAAFPAPIEKTIS
KTKGRPKAPQVYTIPPPKEQMAKDKVSLTCMITDFFPEDITVEWQWNGQP
AENYKNTQPIMDTDGSYFVYSKLNVQKSNWEAGNTFTCSVLHEGLHNHH
TEKSLSHSPGK (SEQ ID NO. 22);
 and/or a light chain variable region comprising or consisting essentially of amino acid sequence: MDFQ V Q IFSFLLISAS VIISRGQ IVL TQSPTIMSVSPGEKVTMTC SAS STISY MHWYQQKSGTSPKRWIYDTSKLASGVPARFSGSGSGTSYSLTITSMGAED AATYYCQQWSSNPPTFGGGTKLEIKRADAAPTVSIFPPSSEQLTSGGASVV CFLNNFYPKDINVKWKIDGSERQNGVLNSWTDQDSKDSTYSMSSTLTLTK DEYERHNSYTCEATHKTSTSPIVKSFNRNEC (SEQ ID NO. 24). The amino acid sequence shown in SEQ ID NO. 24 (also referred to as 129-5 antibody LC) comprises the amino acid sequence shown in SEQ ID NO. 16 and the amino acid sequence shown in SEQ ID NO. 17.

A substance binding to laeverin is, for example, an anti-laeverin antibody (e.g., antigen-binding portion, or Fab, Fab', F(ab')₂, or Fv) comprising a heavy chain variable region comprising or consisting essentially of an amino acid sequence: 
MEWIWIFLFILSGTAGVHSQVQLQQSGAELAGPGTSVRLSCKASGYTFTDY
YIDWVKQRTGQGLEWIGEIYPRSGHSKYNEKFEGKATLTADKSSSTAYMH
LSSLTSEDSAVYFCALYYYGSSHWFAYWGQGTLVTVSAAKTTPPSVYPLA
PGSAAQTNSMVTLGCLVKGYFPEPVTVTWNSGSLSSGVHTFPAVLQSDLY
TLSSSVTVPSSTWPSETVTCNVAHPASSTTVDKKLEPSGPISTINPCPPCKEC
HKCPAPNLEGGPSVFIFPPNIKDVLMISLTPKVTCVVVDVSEDDPDVQISWF
VNNVEVHTAQTQTHREDYNSTIRVVSTLPIQHQDWMSGKEFKCKVNNKD
LPSPIERTISKIKGLVRAPQVYILPPPAEQLSRKDVSLTCLVVGFNPGDISVE
WTSNGHTEENYKDTAPVLDSDGSYFIYSKLNMKTSKWEKTDSFSCNVRHE
GLKNYYLKKTISRSPGK (SEQ ID NO. 25);
 and/or a light chain variable region comprising or consisting essentially of amino acid sequence:

MDSQAQVLMLLLLWVSGTCGDIVMSQSPSSLTVSVGERVTMSCRSSQSLL
YSNIQKNYLAWYQQKPGQSPKLLIYWASTRESGVPDRFTGSGSGTDFTLTI
SSVKAEDLAVYYCQQYYSYPYTFGGGTKLEIKRADAAPTVSIFPPSSEQLT
SGGASVVCFLNNFYPKDINVKWKIDGSERQNGVLNSWTDQDSKDSTYSM
SSTLTLTKDEYERHNSYTCEATHKTSTSPIVKSFNRNEC (SEQ ID NO. 26).

The amino acid sequence shown in SEQ ID NO. 25 (also referred to as 5-23J antibody HC) comprises the amino acid sequence shown in SEQ ID NO. 18 and the amino acid sequence shown in SEQ ID NO. 19. The amino acid sequence shown in SEQ ID NO. 26 (also referred to as ee5-23J antibody LC) comprises the amino acid sequence shown in SEQ ID NO. 20 and the amino acid sequence shown in SEQ ID NO. 21.

A substance binding to laeverin is, for example, a mouse antibody, humanized antibody, or human antibody (e.g., antigen-binding portion or heavy chain variable region) including a heavy chain CDR-H1 (comprising or consisting essentially of the amino acid sequence shown in SEQ ID NO. 1), CDR-H2 (comprising or consisting essentially of the amino acid sequence shown in SEQ ID NO. 2), and CDR -H3 (comprising or consisting essentially of the amino acid sequence shown in SEQ ID NO. 3). A substance binding to laeverin is, for example, a mouse antibody, humanized antibody, or human antibody (e.g., antigen-binding portion or heavy chain variable region) including a light chain CDR-L1 (comprising or consisting essentially of the amino acid sequence shown in SEQ ID NO. 4), CDR-L2 (comprising or consisting essentially of the amino acid sequence ATS), and CDR -L3 (comprising or consisting essentially of the amino acid sequence shown in SEQ ID NO. 5). A substance binding to laeverin is, for example, a mouse antibody, humanized antibody, or human antibody (e.g., antigen-binding portion, or Fab, Fab', F(ab')₂, or Fv) including a heavy chain CDR-H1 (comprising or consisting essentially of the amino acid sequence shown in SEQ ID NO. 1), CDR-H2 (comprising or consisting essentially of the amino acid sequence shown in SEQ ID NO. 2), and CDR -H3 (comprising or consisting essentially of the amino acid sequence shown in SEQ ID NO. 3), and a light chain CDR-L1 (comprising or consisting essentially of the amino acid sequence shown in SEQ ID NO. 4), CDR-L2 (comprising or consisting essentially of the amino acid sequence ATS), and CDR-L3 (comprising or consisting essentially of the amino acid sequence shown in SEQ ID NO. 5).

A substance binding to laeverin is, for example, a mouse antibody, humanized antibody, or human antibody (e.g., antigen-binding portion or heavy chain variable region) including a heavy chain CDR-H1 (comprising or consisting essentially of the amino acid sequence shown in SEQ ID NO. 11), CDR-H2 (comprising or consisting essentially of the amino acid sequence shown in SEQ ID NO. 12), and CDR -H3 (comprising or consisting essentially of the amino acid sequence shown in SEQ ID NO. 13). A substance binding to laeverin is, for example, a mouse antibody, humanized antibody, or human antibody (e.g., antigen-binding portion or heavy chain variable region) including a light chain CDR-L1 (comprising or consisting essentially of the amino acid sequence shown in SEQ ID NO. 14 or 16), CDR-L2 (comprising or consisting essentially of the amino acid sequence ATS or WAS), and CDR-L3 (comprising or consisting essentially of the amino acid sequence shown in SEQ ID NO. 15 or 17). A substance binding to laeverin is, for example, a mouse antibody, humanized antibody, or human antibody (e.g., antigen-binding portion, or Fab, Fab', F(ab')₂ , or Fv) including a heavy chain CDR-H1 (comprising or consisting essentially of the amino acid sequence shown in SEQ ID NO. 11), CDR-H2 (comprising or consisting essentially of the amino acid sequence shown in SEQ ID NO. 12), and CDR -H3 (comprising or consisting essentially of the amino acid sequence shown in SEQ ID NO. 13), and a light chain CDR-L1 (comprising or consisting essentially of the amino acid sequence shown in SEQ ID NO. 14), CDR-L2 (comprising or consisting essentially of the amino acid sequence ATS), and CDR-L3 (comprising or consisting essentially of the amino acid sequence shown in SEQ ID NO. 15). A substance binding to laeverin is, for example, a mouse antibody, humanized antibody, or human antibody (e.g., antigen-binding portion, or Fab, Fab', F(ab')₂, or Fv) including a heavy chain CDR-H1 (comprising or consisting essentially of the amino acid sequence shown in SEQ ID NO. 11), CDR-H2 (comprising or consisting essentially of the amino acid sequence shown in SEQ ID NO. 12), and CDR -H3 (comprising or consisting essentially of the amino acid sequence shown in SEQ ID NO. 13), and a light chain CDR-L1 (comprising or consisting essentially of the amino acid sequence shown in SEQ ID NO. 16), CDR-L2 (comprising or consisting essentially of the amino acid sequence DTS), and CDR-L3 (comprising or consisting essentially of the amino acid sequence shown in SEQ ID NO. 17).

A substance binding to laeverin is, for example, a mouse antibody, humanized antibody, or human antibody (e.g., antigen-binding portion or heavy chain variable region) including a heavy chain CDR-H1 (comprising or consisting essentially of the amino acid sequence shown in SEQ ID NO. 18) and CDR-H2 (comprising or consisting essentially of the amino acid sequence shown in SEQ ID NO. 19). A substance binding to laeverin is, for example, a mouse antibody, humanized antibody, or human antibody (e.g., antigen-binding portion or heavy chain variable region) including a heavy chain CDR-L1 (comprising or consisting essentially of the amino acid sequence shown in SEQ ID NO. 20), CDR-L2 (comprising or consisting essentially of the amino acid sequence WAS), and CDR-L3 (comprising or consisting essentially of the amino acid sequence shown in SEQ ID NO. 21). A substance binding to laeverin is, for example, a mouse antibody, humanized antibody, or human antibody (e.g., antigen-binding portion, or Fab, Fab', F(ab')₂, or Fv) including a heavy chain CDR-H1 (comprising or consisting essentially of the amino acid sequence shown in SEQ ID NO. 18) and CDR-H2 (comprising or consisting essentially of the amino acid sequence shown in SEQ ID NO. 19), and a light chain CDR-L1 (comprising or consisting essentially of the amino acid sequence shown in SEQ ID NO. 20), CDR-L2 (comprising or consisting essentially of the amino acid sequence WAS), and CDR-L3 (comprising or consisting essentially of the amino acid sequence shown in SEQ ID NO. 21).

A substance binding to laeverin is, for example, an anti-laeverin antibody (e.g,, mouse antibody, humanized antibody, or human antibody) including a heavy chain comprising or consisting essentially of an amino acid sequence having at least 70% (e.g., 75% or more, 80% or more, 85% or more, preferably 90% or more, more preferably 95% or more) sequence identity to the amino acid sequence shown in SEQ ID NO. 6, and/or or a light chain comprising or consisting essentially of an amino acid sequence having at least 70% (e.g., 75% or more, 80% or more, 85% or more, preferably 90% or more, more preferably 95% or more) sequence identity to the amino acid sequence shown in SEQ ID NO. 7, wherein the heavy chain comprises CDR-H1 comprising or consisting of the amino acid sequence shown in SEQ ID NO. 1, CDR-H2 comprising or consisting of the amino acid sequence shown in SEQ ID NO. 2, and comprising or consisting of the amino acid sequence shown in SEQ ID NO. 3, and the light chain comprises CDR-L1 comprising or consisting of the amino acid sequence shown in SEQ ID NO. 4, CDR-L2 comprising or consisting of the amino acid sequence ATS, and CDR-L3 comprising or consisting of the amino acids shown in SEQ ID NO. 5.

A substance binding to laeverin is, for example, an anti-laeverin antibody (e.g., antigen-binding portion, or Fab, Fab', F(ab')₂, or Fv) including a heavy chain variable region encoded by the nucleotide sequence: 
ATGTCCTCTCCACAGCCCCTGAAGACACTGACTCTAACCATGGGAGGG
AGCTGGATCTTTCTCTTCCTCCTGTCAGGAACTGCAGGTGCCCACTCTG
AGATCCAGCTGCAGCAGACTGGACCTGAGCTGGTGAAGCCTGGGGCTT
CAGTGAAGATATCCTGCAAGGCTTCCGGTTATTCATTCACTGACTACAT
CATGCTCTGGGTGAAGCAGAGCCATGGAAAGAGCCTTGAGTGGATTGG
AAATATTAATCCTTACCATGCTGGTATTAGCTACAATCTGAAGTTCAAG
GGCAAGGCCACATTGACTGTAGACACATCTTCCAGCACAGCCTACATG
CAGCTCAACAGTCTGACATCTGAGGACTCTGCAGTCTATTACTGTGCAA
GAGGGAGTAATTACGTCTATTACTATGCTATGGACTACTGGGGTCAAG
GAACCTCAGTCACCGTCTCCTCAGCCAAAACGACACCCCCATCTGTCTA
TCCACTGGCCCCTGGATCTGCTGCCCAAACTAACTCCATGGTGACCCTG
GGATGCCTGGTCAAGGGCTATTTCCCTGAGCCAGTGACAGTGACCTGG
AACTCTGGATCCCTGTCCAGCGGTGTGCACACCTTCCCAGCTGTCCTGC
AGTCTGACCTCTACACTCTGAGCAGCTCAGTGACTGTCCCCTCCAGCAC
CTGGCCCAGCGAGACCGTCACCTGCAACGTTGCCCACCCGGCCAGCAG
CACCAAGGTGGACAAGAAAATTGTGCCCAGGGATTGTGGTTGTAAGCC
TTGCATATGTACAGTCCCAGAAGTATCATCTGTCTTCATCTTCCCCCCA
AAGCCCAAGGATGTGCTCACCATTACTCTGACTCCCAAGGTCACGTGTG
TTGTGGTAGACATCAGCAAGGATGATCCCGAGGTCCAGTTCAGCTGGT
TTGTAGATGATGTGGAGGTGCACACAGCTCAGACGCAACCCCGGGAGG
AGCAGTTCAACAGCACTTTCCGCTCAGTCAGTGAACTTCCCATCATGCA
CCAGGACTGGCTCAATGGCAAGGAGTTCAAATGCAGGGTCAACAGTGC
AGCTTTCCCTGCCCCCATCGAGAAAACCATCTCCAAAACCAAAGGCAG
ACCGAAGGCTCCACAGGTGTACACCATTCCACCTCCCAAGGAGCAGAT
GGCCAAGGATAAAGTCAGTCTGACCTGCATGATAACAGACTTCTTCCCT
 
GAAGACATTACTGTGGAGTGGCAGTGGAATGGGCAGCCAGCGGAGAA
CTACAAGAACACTCAGCCCATCATGGACACAGATGGCTCTTACTTCGTC
TACAGCAAGCTCAATGTGCAGAAGAGCAACTGGGAGGCAGGAAATACT
TTCACCTGCTCTGTGTTACATGAGGGCCTGCACAACCACCATACTGAGA
AGAGCCTCTCCCACTCTCCTGGTAAA (SEQ ID NO. 8),
 and/or a light chain variable region encoded by the nucleotide sequence: ATGGATTTTCAAGTGCAGATTTTCAGCTTCCTGCTAATCAGTGCTTCAG TCATAATGTCCAGAGGACAAATTGTTCTCTCCCAGTCTCCAGCAATTCT GTCTGCATCTCCAGGGGAGAAGGTCACAATGACTTGCAGGGCCAGTTC AAGTGTAAGTTACATGCACTGGTATCAGCAGAAGCCAGGATCCTCCCC CAAACCCTGGATTTTTGCCACATCCAACCTGGCTTCTGGAGTCCCTGCT CGCTTCAGTGGCAGTGGGTCTGGGACCTCTTATTCTCTCACAATCAGCA GAGTGGAGGCTGAAGATGCTGCCACTTATTACTGCCAGCAGTGGAGTA GTAACCCACCGACGTTCGGTGGAGGCACCAAGCTGGAAATCAAACGGG CTGATGCTGCACCAACTGTATCCATCTTCCCACCATCCAGTGAGCAGTT AACATCTGGAGGTGCCTCAGTCGTGTGCTTCTTGAACAACTTCTACCCC AAAGACATCAATGTCAAGTGGAAGATTGATGGCAGTGAACGACAAAAT GGCGTCCTGAACAGTTGGACTGATCAGGACAGCAAAGACAGCACCTAC AGCATGAGCAGCACCCTCACGTTGACCAAGGACGAGTATGAACGACAT AACAGCTATACCTGTGAGGCCACTCACAAGACATCAACTTCACCCATT GTCAAGAGCTTCAACAGGAATGAGTGT (SEQ ID NO. 9). The nucleotide sequence shown in SEQ ID NO. 8 encodes the amino acid sequence shown in SEQ ID NO. 6. The nucleotide sequence shown in SEQ ID NO. 9 encodes the amino acid sequence shown in SEQ ID NO. 7.

A substance binding to laeverin is, for example, an anti-laeverin antibody (e.g., antigen-binding portion, or Fab, Fab', F(ab')₂, or Fv) including a heavy chain variable region encoded by the nucleotide 
sequence:ATGGGATGGAGCTATATCATCCTCTTCTTGTTAGCAACAGCTA
CATGTGTCCACTCCCAGGTCCAACTGCAGCAGCCTGGGGCTGAGCTGG
TGAGGCCTGGGGCTTCAGTGAAGCTGTCCTGCAAGGCTTCTGGCTACAC
GTTCACCAGCTACTGGATGAACTGGGTTAGGCAGAGGCCTGAGCAAGG
 
CCTTGAGTGGATTGGAAGGATTGATCCTTACGATAGTGAAACTCACTAC
AATCAAAAGTTCAAGGACAAGGCCATTTTGACTGTAGACAAATCCTCC
AGTACAGTCCACATGCAACTAAGCAGCCTGACATCTGAGGACTCTGCG
GTCTATCACTGTGCAAGAGACTACGGTAGTAGGTACTATGCTATGGACT
ACTGGGGTCAAGGAACCTCAGTCACCGTCTCCTCAGCCAAAACGACAC
CCCCATCTGTCTATCCACTGGCCCCTGGATCTGCTGCCCAAACTAACTC
CATGGTGACCCTGGGATGCCTGGTCAAGGGCTATTTCCCTGAGCCAGTG
ACAGTGACCTGGAACTCTGGATCCCTGTCCAGCGGTGTGCACACCTTCC
CAGCTGTCCTGCAGTCTGACCTCTACACTCTGAGCAGCTCAGTGACTGT
CCCCTCCAGCACCTGGCCCAGCGAGACCGTCACCTGCAACGTTGCCCA
CCCGGCCAGCAGCACCAAGGTGGACAAGAAAATTGTGCCCAGGGATTG
TGGTTGTAAGCCTTGCATATGTACAGTCCCAGAAGTATCATCTGTCTTC
ATCTTCCCCCCAAAGCCCAAGGATGTGCTCACCATTACTCTGACTCCCA
AGGTCACGTGTGTTGTGGTAGACATCAGCAAGGATGATCCCGAGGTCC
AGTTCAGCTGGTTTGTAGATGATGTGGAGGTGCACACAGCTCAGACGC
AACCCCGGGAGGAGCAGTTCAACAGCACTTTCCGCTCAGTCAGTGAAC
TTCCCATCATGCACCAGGACTGGCTCAATGGCAAGGAGTTCAAATGCA
GGGTCAACAGTGCAGCTTTCCCTGCCCCCATCGAGAAAACCATCTCCA
AAACCAAAGGCAGACCGAAGGCTCCACAGGTGTACACCATTCCACCTC
CCAAGGAGCAGATGGCCAAGGATAAAGTCAGTCTGACCTGCATGATAA
CAGACTTCTTCCCTGAAGACATTACTGTGGAGTGGCAGTGGAATGGGC
AGCCAGCGGAGAACTACAAGAACACTCAGCCCATCATGGACACAGATG
GCTCTTACTTCGTCTACAGCAAGCTCAATGTGCAGAAGAGCAACTGGG
AGGCAGGAAATACTTTCACCTGCTCTGTGTTACATGAGGGCCTGCACA
ACCACCATACTGAGAAGAGCCTCTCCCACTCTCCTGGTAAATGA (SEQ
ID NO. 27),
 and/or a light chain variable region encoded by the nucleotide sequence:ATGGGCATCAAGATGGAATCACAGACTCTGGTCTTCATATCCA TACTGCTCTGGTTATATGGAGCTGATGGGAACATTGTAATGACCCAATC TCCCAAATCCATGTCCATGTCAGTAGGAGAGAGGGTCACCTTGACCTG CAAGGCCAGTGAGAATGTGGTTACTTATGTTTCCTGGTATCAACAGAA ACCAGAGCAGTCTCCTAAACTGCTGATATACGGGGCATCCAACCGGTA CACTGGGGTCCCCGATCGCTTCACAGGCAGTGGATCTGCAACAGATTTC ACTCTGACCATCAGCAGTGTGCAGGCTGAAGACCTTGCAGATTATCACT GTGGACAGGGTTACAGCTATCCGTACACGTTCGGAGGGGGGACCAAGC TGGAAATAAAACGGGCTGATGCTGCACCAACTGTATCCATCTTCCCACC ATCCAGTGAGCAGTTAACATCTGGAGGTGCCTCAGTCGTGTGCTTCTTG AACAACTTCTACCCCAAAGACATCAATGTCAAGTGGAAGATTGATGGC AGTGAACGACAAAATGGCGTCCTGAACAGTTGGACTGATCAGGACAGC AAAGACAGCACCTACAGCATGAGCAGCACCCTCACGTTGACCAAGGAC GAGTATGAACGACATAACAGCTATACCTGTGAGGCCACTCACAAGACA TCAACTTCACCCATTGTCAAGAGCTTCAACAGGAATGAGTGTTAG (SEQ ID NO. 28). The nucleotide sequence shown in SEQ ID NO. 27 encodes the amino acid sequence shown in SEQ ID NO. 22. The nucleotide sequence shown in SEQ ID NO. 28 encodes the amino acid sequence shown in SEQ ID NO. 23.

A substance binding to laeverin is, for example, an anti-laeverin antibody (e.g., antigen-binding portion, or Fab, Fab', F(ab')₂, or Fv) including a heavy chain variable region encoded by the nucleotide sequence: 
ATGGGATGGAGCTATATCATCCTCTTCTTGTTAGCAACAGCTACATGTG
TCCACTCCCAGGTCCAACTGCAGCAGCCTGGGGCTGAGCTGGTGAGGC
CTGGGGCTTCAGTGAAGCTGTCCTGCAAGGCTTCTGGCTACACGTTCAC
CAGCTACTGGATGAACTGGGTTAGGCAGAGGCCTGAGCAAGGCCTTGA
GTGGATTGGAAGGATTGATCCTTACGATAGTGAAACTCACTACAATCA
AAAGTTCAAGGACAAGGCCATTTTGACTGTAGACAAATCCTCCAGTAC
AGTCCACATGCAACTAAGCAGCCTGACATCTGAGGACTCTGCGGTCTA
TCACTGTGCAAGAGACTACGGTAGTAGGTACTATGCTATGGACTACTG
GGGTCAAGGAACCTCAGTCACCGTCTCCTCAGCCAAAACGACACCCCC
ATCTGTCTATCCACTGGCCCCTGGATCTGCTGCCCAAACTAACTCCATG
GTGACCCTGGGATGCCTGGTCAAGGGCTATTTCCCTGAGCCAGTGACA
GTGACCTGGAACTCTGGATCCCTGTCCAGCGGTGTGCACACCTTCCCAG
CTGTCCTGCAGTCTGACCTCTACACTCTGAGCAGCTCAGTGACTGTCCC
CTCCAGCACCTGGCCCAGCGAGACCGTCACCTGCAACGTTGCCCACCC
GGCCAGCAGCACCAAGGTGGACAAGAAAATTGTGCCCAGGGATTGTGG
 
TTGTAAGCCTTGCATATGTACAGTCCCAGAAGTATCATCTGTCTTCATC
TTCCCCCCAAAGCCCAAGGATGTGCTCACCATTACTCTGACTCCCAAGG
TCACGTGTGTTGTGGTAGACATCAGCAAGGATGATCCCGAGGTCCAGT
TCAGCTGGTTTGTAGATGATGTGGAGGTGCACACAGCTCAGACGCAAC
CCCGGGAGGAGCAGTTCAACAGCACTTTCCGCTCAGTCAGTGAACTTC
CCATCATGCACCAGGACTGGCTCAATGGCAAGGAGTTCAAATGCAGGG
TCAACAGTGCAGCTTTCCCTGCCCCCATCGAGAAAACCATCTCCAAAAC
CAAAGGCAGACCGAAGGCTCCACAGGTGTACACCATTCCACCTCCCAA
GGAGCAGATGGCCAAGGATAAAGTCAGTCTGACCTGCATGATAACAGA
CTTCTTCCCTGAAGACATTACTGTGGAGTGGCAGTGGAATGGGCAGCC
AGCGGAGAACTACAAGAACACTCAGCCCATCATGGACACAGATGGCTC
TTACTTCGTCTACAGCAAGCTCAATGTGCAGAAGAGCAACTGGGAGGC
AGGAAATACTTTCACCTGCTCTGTGTTACATGAGGGCCTGCACAACCAC
CATACTGAGAAGAGCCTCTCCCACTCTCCTGGTAAATGA (SEQ ID NO.
27),
 and/or a light chain variable region encoded by the nucleotide sequence: 
ATGGATTTTCAAGTGCAGATTTTCAGCTTCCTGCTAATCAGTGCCTCAG
TCATAATATCCAGAGGACAAATTGTTCTCACCCAGTCTCCAACAATCAT
GTCTGTATCTCCAGGGGAGAAGGTCACCATGACCTGCAGTGCCAGCTC
AACTATAAGTTACATGCACTGGTACCAGCAGAAGTCAGGCACCTCCCC
CAAAAGATGGATTTATGACACATCCAAACTGGCTTCTGGAGTCCCTGCT
CGCTTCAGTGGCAGTGGGTCTGGGACCTCTTACTCTCTCACAATCACCA
GCATGGGGGCTGAAGATGCTGCCACTTATTACTGCCAGCAGTGGAGTA
GTAACCCACCCACGTTCGGAGGGGGGACCAAGCTGGAAATAAAACGG
GCTGATGCTGCACCAACTGTATCCATCTTCCCACCATCCAGTGAGCAGT
TAACATCTGGAGGTGCCTCAGTCGTGTGCTTCTTGAACAACTTCTACCC
CAAAGACATCAATGTCAAGTGGAAGATTGATGGCAGTGAACGACAAAA
TGGCGTCCTGAACAGTTGGACTGATCAGGACAGCAAAGACAGCACCTA
CAGCATGAGCAGCACCCTCACGTTGACCAAGGACGAGTATGAACGACA
TAACAGCTATACCTGTGAGGCCACTCACAAGACATCAACTTCACCCATT
GTCAAGAGCTTCAACAGGAATGAGTGTTAG (SEQ ID NO. 29).
 The nucleotide sequence shown in SEQ ID NO. 29 encodes the amino acid sequence shown in SEQ ID NO. 24.

A substance binding to laeverin is, for example, an anti-laeverin antibody (e.g., antigen-binding portion, or Fab, Fab', F(ab')₂, or Fv) including a heavy chain variable region encoded by the nucleotide sequence : 
:ATGGAATGGATCTGGATCTTTCTCTTCATCCTCTCAGGAACT
GCAGGTGTCCACTCCCAGGTTCAGCTGCAGCAGTCTGGAGCTGAGCTG
GCGGGGCCCGGGACTTCAGTGAGGCTGTCCTGCAAGGCTTCTGGCTAC
ACCTTCACTGACTACTACATAGACTGGGTGAAGCAGAGGACTGGACAG
GGCCTTGAGTGGATTGGAGAGATTTATCCTAGAAGTGGTCATTCTAAGT
ACAACGAGAAGTTCGAGGGCAAGGCCACACTGACTGCAGACAAATCCT
CCAGCACAGCCTACATGCACCTCAGCAGCCTGACATCTGAAGACTCTG
CAGTCTATTTCTGTGCCCTCTATTACTACGGCAGTAGTCACTGGTTTGCT
TACTGGGGCCAAGGGACTCTGGTCACTGTCTCTGCAGCCAAAACGACA
CCCCCATCTGTCTATCCACTGGCCCCTGGATCTGCTGCCCAAACTAACT
CCATGGTGACCCTGGGATGCCTGGTCAAGGGCTATTTCCCTGAGCCAGT
GACAGTGACCTGGAACTCTGGATCCCTGTCCAGCGGTGTGCACACCTTC
CCAGCTGTCCTGCAGTCTGACCTCTACACTCTGAGCAGCTCAGTGACTG
TCCCCTCCAGCACCTGGCCCAGCGAGACCGTCACCTGCAACGTTGCCCA
CCCGGCCAGCAGCACCACGGTGGACAAAAAACTTGAGCCCAGCGGGCC
CATTTCAACAATCAACCCCTGTCCTCCATGCAAGGAGTGTCACAAATGC
CCAGCTCCTAACCTCGAGGGTGGACCATCCGTCTTCATCTTCCCTCCAA
ATATCAAGGATGTACTCATGATCTCCCTGACACCCAAGGTCACGTGTGT
GGTGGTGGATGTGAGCGAGGATGACCCAGACGTCCAGATCAGCTGGTT
TGTGAACAACGTGGAAGTACACACAGCTCAGACACAAACCCATAGAGA
GGATTACAACAGTACTATCCGGGTGGTCAGCACCCTCCCCATCCAGCA
CCAGGACTGGATGAGTGGCAAGGAGTTCAAATGCAAGGTCAACAACAA
AGACCTCCCATCACCCATCGAGAGAACCATCTCAAAAATTAAAGGGCT
AGTCAGAGCTCCACAAGTATACATCTTGCCGCCACCAGCAGAGCAGTT
GTCCAGGAAAGATGTCAGTCTCACTTGCCTGGTCGTGGGCTTCAACCCT
GGAGACATCAGTGTGGAGTGGACCAGCAACGGGCATACAGAGGAGAA
CTACAAGGACACCGCACCAGTCCTGGACTCTGACGGTTCTTACTTCATA
 
TATAGCAAGCTCAATATGAAAACAAGCAAGTGGGAGAAAACAGATTCC
TTCTCATGCAACGTGAGACACGAGGGTCTGAAAAATTACTACCTGAAG
AAGACCATCTCCCGGTCTCCGGGTAAATGA (SEQ ID NO. 30),
 and/or a light chain variable region encoded by the nucleotide sequence: ATGGATTCACAGGCCCAGGTTCTTATGTTACTGCTGCTATGGGTATCTG GTACCTGTGGGGACATTGTGATGTCACAGTCTCCATCCTCCCTAACTGT GTCAGTTGGAGAGAGGGTTACTATGAGCTGCAGGTCCAGTCAGAGCCT TTTATATAGTAACATTCAAAAGAACTACTTGGCCTGGTACCAGCAGAA ACCGGGGCAGTCTCCTAAACTGCTGATTTACTGGGCATCCACTAGGGA ATCTGGGGTCCCTGATCGCTTCACAGGCAGTGGATCTGGGACAGATTTC ACTCTCACCATCAGCAGTGTGAAGGCTGAAGACCTGGCAGTTTATTACT GTCAGCAATATTATAGCTATCCGTACACGTTCGGAGGGGGGACCAAGC TGGAAATAAAACGGGCTGATGCTGCACCAACTGTATCCATCTTCCCACC ATCCAGTGAGCAGTTAACATCTGGAGGTGCCTCAGTCGTGTGCTTCTTG AACAACTTCTACCCCAAAGACATCAATGTCAAGTGGAAGATTGATGGC AGTGAACGACAAAATGGCGTCCTGAACAGTTGGACTGATCAGGACAGC AAAGACAGCACCTACAGCATGAGCAGCACCCTCACGTTGACCAAGGAC GAGTATGAACGACATAACAGCTATACCTGTGAGGCCACTCACAAGACA TCAACTTCACCCATTGTCAAGAGCTTCAACAGGAATGAGTGTTAG (SEQ ID NO. 31). The nucleotide sequence shown in SEQ ID NO. 30 encodes the amino acid sequence shown in SEQ ID NO. 25. The nucleotide sequence shown in SEQ ID NO. 31 encodes the amino acid sequence shown in SEQ ID NO. 26.

A "cytotoxic agent" comprises a substance capable of inhibiting or arresting cell growth or kill or destroy cells. Cytotoxic agent may be, for example, a radioisotope (e.g., At²¹¹, I¹³¹, I¹²⁵, Y⁹⁰, Re¹⁸⁶, Re¹⁸⁸, Sm¹⁵³, Bi²¹², P³², Pb²¹², or radioisotope of Lu); a chemotherapeutic agent (e.g., monomethyl auristatin E (MMAE), methotrexate, gemcitabine, adriamycin, vinca alkaloids, cisplatin, ifomide, dacarbazine, carboplatin, bleomycin, paclitaxel, carboplatin, vinorelbine, irinotecan (CPT-11), doxorubicin, melphalan, mitomycin C, chlorambucil, daunorubicin, erlotinib or other intercalating agent or growth inhibitor); an enzyme, such as a nucleolytic enzyme, or a fragment thereof; toxin (e.g., a small molecule toxin or enzyme-active toxin originated from bacterial, fungal, plant or animal, or a fragment and/or mutant thereof); or a combination thereof.

A "cytotoxic agent-conjugated substance binding to laeverin" can be produced, for example, by conjugating a substance binding to laeverin with a cytotoxic agent. The conjugation can be, for example, a direct or indirect conjugation. Direct conjugation is, for example, a covalent bond. Direct conjugation is formed, for example, by a method utilizing periodate, glutaraldehyde, maleimide, or N-hydroxysuccinimide. Direct conjugation of a substance binding to laeverin to a cytotoxic agent includes, for example, the covalent bonding of the substance binding to laeverin and the cytotoxic agent via a linker. Indirect conjugation is, for example, non-covalent bonding. Indirect conjugation is formed, for example, via biotin-streptavidin binding.

A cytotoxic agent-conjugated substance binding to laeverin includes, for example, at least one anti-laeverin antibody and at least one (e.g., one, two, or three or more) cytotoxic agent selected from the group consisting of radioisotopes and chemotherapeutic agents, enzymes or fragments thereof, and toxins. A cytotoxic agent-conjugated substance binding to laeverin includes, for example, one substance binding to laeverin and one cytotoxic agent selected from the group consisting of radioisotopes, chemotherapeutic agents, enzymes or fragments thereof, and toxins. A cytotoxic agent-conjugated substances binding to laeverin includes, for example, an anti-laeverin antibody and a chemotherapeutic agent or radioisotope or a combination thereof. A cytotoxic agent-conjugated substance binding to laeverin includes, for example, an anti-laeverin antibody and a chemotherapeutic agent.

A cytotoxic agent-conjugated substance binding to laeverin includes, for example, one substance binding to laeverin and at least one (e.g., one, two, or three or more) cytotoxic agent. A cytotoxic agent-conjugated substance binding to laeverin includes, for example, one anti-laeverin antibody and a plurality of one cytotoxic agent selected from the group consisting of radioisotopes, chemotherapeutic agents, enzymes or fragments thereof, and toxins. A cytotoxic agent-conjugated substance binding to laeverin includes, for example, one anti-laeverin antibody and a plurality of chemotherapeutic agents, radioisotopes, or combinations thereof. A cytotoxic agent-conjugated substance binding to laeverin includes, for example, one anti-laeverin antibody and multiple or one chemotherapeutic agent.

The term "cancer cell" refers to a cell possessing uncontrolled cell growth and/or invasive characteristics. For example, cancer cell proliferates in a scaffold-dependent or scaffold-independent manner. Cancer cells can form a floating cell aggregate(s) composed of multiple cells. Cancer cells are, for example, circulating tumor cells (CTCs). The term "circulating tumor cell" or "CTC" refers to a cancer cell that are detached from primary tumor tissue or metastatic tumor tissue and circulates in the bloodstream. Cancer cell is, for example, a cancer cell expressing laeverin existing in a blood vessel or in a lymphatic vessel.

The term "IDO1" or "indoleamine 2,3-dioxygenase-1" refers to an enzyme involved in a rate limitation of the kynurenine pathway in which tryptophan is metabolized into kynurenine (see Folia Pharmacol. Jpn. 142, 85-88 (2013)). When IDO1 is highly expressed in tumor cells, T cells and NK cells around the tumor cells are inhibited in their proliferation or lead to apoptotic (see Folia Pharmacol. Jpn. 142, 85-88 (2013)). Differentiation into regulatory T cell (Treg) is induced. Tregs are accumulated around the environment surrounding IDO1-expressing cancer cells, resulting in the support of their survival (Liu et al., Targeting the IDO1 pathway in cancer: from bench to bedside. Journal of Hematology & Oncology (2018)11:100). In a population of subjects underwent the treatment of squamous cell carcinoma of the head and neck, the subject group in which mRNA encoding IDO1 was detected in blood CTCs had a shorter overall survival than the subject group in which no such mRNA was detected (Economopoulou P, et. al., Prognostic impact of indoleamine 2,3-dioxygenase 1 <i>(IDO1)</i> mRNA expression on circulating tumour cells of patients with head and neck squamous cell carcinoma. ESMO Open. 2020 May;5(3):e000646. doi: 10.1136/esmoopen-2019-000646. PMID: 32414944; PMCID: PMC7232623.). IDO1 can be measured, for example, by detecting or quantifying mRNA encoding IDO1. The mRNA can be detected or quantified, for example, by quantitative PCR using reverse transcriptase (RT-qPCR).

Examples in the present disclosure demonstrate that CTCs expressing laeverin were present in blood samples from patients with recurrent and/or metastatic cancer. The present disclosure also describes that the CTCs expressed indoleamine 2,3-dioxygenase-1 (IDO1). A cancer that may be treated with a pharmaceutical composition according to the present disclosure is, for example, a cancer in a subject in which a cancer cell (e.g., CTC) expressing laeverin is detected. A cancers that may be treated with a pharmaceutical composition according to the present disclosure is, for example, a cancer in a subject in which cancer cells (e.g., CTCs and cancer cells floating in lymphatic vessels) expressing laeverin and IDO1 are detected. A cancer that may be treated with a pharmaceutical composition according to the present disclosure is, for example, a cancer after treatment with chemotherapy and/or radiation therapy.

Examples in the present disclosure suggest that cancer cells expressing laeverin form a tumor microenvironment (TME), due to IDO1 expressed by themselves and by IDO1 expressed in differentiated dendritic cells induced from monocytes around the cancer cells. The TME allows the cancer cells to evade immune system attacks. Examples in the present disclosure suggest that a substance binding to laeverin can inhibit the expression of IDO1 in the cancer cells and prevent the differentiation of monocytes into IDOl-expressing dendritic cells through the contact with the cancer cells. The binding of a substance binding to laeverin on the cell membrane of cancer cells, for example, its steric hindrance due to the binding can inhibit the formation of TMEs that allow the cancer cells to evade immune system attacks, thereby the cancer cells attacked by the immune system. Thus, a substance binding to laeverin can be used in the production of a pharmaceutical composition for treating cancer.

The term "cancer" refers to a broad range of tumors. Tumor includes, for example, lung cancer, prostate cancer, breast cancer, mammary gland cancer, pancreatic cancer, stomach cancer, rectal cancer, colon cancer, colorectal cancer, cancer of the thyroid gland, liver cancer, gall cancer, glioma, glioblastoma, uterine cancer, cervical cancer, kidney cancer, ovarian cancer, fallopian tube cancer, esophageal cancer, melanoma, sarcoma, blood cancer, basal cell cancer, and squamous cell carcinoma. Examples in the present disclosure demonstrate that cancer cells expressing or capable of expressing laeverin (e.g., under sever conditions for cell survival, such as floating in lymphatics or in the presence of chemotherapeutic agents) play an essential role in cancer metastasis (e.g., systemic metastasis) and/or recurrence (e.g., local relapse). A cancer that may be prevented or treated by a pharmaceutical composition according to the present disclosure is, for example, recurrent, metastatic, or resistant cancer.

Examples in the present disclosure demonstrate that placental-site trophoblast tumors express laeverin. Examples in the present disclosure also demonstrate that B cells and monocytes among peripheral blood mononuclear cells express laeverin. A cancer that may be prevented or treated by a pharmaceutical composition according to the present disclosure is, for example, choriocarcinoma, placental trophoblast tumor, ovarian cancer, fallopian tube cancer, uterine cancer, cervical cancer, glioblastoma, colon cancer, prostate cancer, or leukemia. The cancer is, for example, choriocarcinoma, placental trophoblast tumor, ovarian cancer, fallopian tube cancer, uterine cancer, cervical cancer, or leukemia. The cancer is, for example, choriocarcinoma, placental trophoblast tumor, ovarian cancer, fallopian tube cancer, uterine cancer, or cervical cancer. The cancer is, for example, choriocarcinoma or placental trophoblast tumor. A pharmaceutical composition according to the present disclosure includes, for example, a substance binding to laeverin to which a cytotoxic agent may be conjugated (e.g., MMAE-conjugated anti-laeverin antibody).

Examples in the present disclosure demonstrate that an anti-laeverin antibody (without a cytotoxic agent) induces cell death in ovarian cancer cells. Examples in the present disclosure also demonstrate that an anti-laeverin antibody (without cytotoxic agents) does not induce cell death to cell death in breast cancer cell lines and melanoma. Cancer that may be prevented or treated with a pharmaceutical composition comprising a substance binding to laeverin without conjugation of a cytotoxic agent is, for example, choriocarcinoma, placental trophoblast tumor, ovarian cancer, fallopian tube cancer, uterine cancer, cervical cancer, glioblastoma, colon cancer, prostate cancer, or leukemia. The cancer is, for example, choriocarcinoma, placental trophoblast tumor, ovarian cancer, fallopian tube cancer, uterine cancer, or cervical cancer. The cancer is, for example, ovarian, fallopian tube, uterine, or cervical cancer.

"Cancer recurrence" or "recurrent cancer" refers to a growth or a possible growth of a cancer cell at either or both the primary tumor site and a distal site after the cancer responded to a treatment including chemotherapy and/or surgery. Locally recurrent cancer refers to a growth or a possible growth of a cancer cell at the primary tumor site after response to the treatment. "Metastasis" or "metastatic cancer" refers to a spreading or possible spreading of cancer cell from one portion of the body (e.g., uterus) to another portion of the body (e.g., lungs). Recurrent or metastatic cancer is, for example, cancer that is identified as being at risk of recurrence or metastasis or cancer recurred or metastasized according to known criteria. Metastatic cancer may be cancer after the treatment including surgically, or untreated.

"Resistant cancer" means that cancer cells grow or are likely to grow in a subject during a cancer therapy including chemotherapy or biologic therapy such as immunotherapy. "Cancer therapy" includes the use of a therapeutic agent (e.g., radioisotope and chemotherapeutic agent (including immunotherapeutic agent)), radiation therapy, and surgical interventions such as surgery in the treatment of cancer in a mammal.

A "pharmaceutical composition" is a mixture comprising at least one active ingredient for preventing or treating cancer and a pharmaceutically acceptable carrier. A pharmaceutical composition according to the present disclosure comprises, for example, a substance binding to laeverin according to the present disclosure and a pharmaceutically acceptable carrier. The pharmaceutical composition may be combined with another active ingredient (e.g., chemotherapeutic agent) for preventing or treating cancer to the extent that the effect of the substance binding to laeverin of the present disclosure is not impaired. The combination includes administering the additional active ingredient before, after, or simultaneously with administration of the substance binding to laeverin of the present disclosure. When the cancer is a resistant cancer, the pharmaceutical composition according to the present disclosure may be administered after or during a cancer therapy such as chemotherapy.

The pharmaceutical composition may be, for example, in solid or liquid form. The pharmaceutical composition may be, for example, a capsule, a tablet, a powder, a liquid, a suspension, an injection, or a suppository. The pharmaceutical composition may be prepared according to known methods. For example, the pharmaceutical composition can be prepared by mixing a substance binding to laeverin according to the present disclosure with a pharmaceutically acceptable carrier. The pharmaceutical composition according to the present disclosure can be administered to a subject by a known method of administration. Known methods of administration include, for example, oral and parenteral administration. Parenteral administration includes, for example, intramuscular, intraperitoneal, intravenous, and subcutaneous administration.

"Treat" or "treatment" includes eliminating, reducing, or alleviating at least one symptom, prolonging overall survival, prolonging duration of response, slowing disease progression, or preventing or delaying the onset of symptoms in a subject. Treatment of cancer includes, for example, alleviation or complete eradication of cancer symptoms. Treatment of cancer includes, for example, prevention or therapy of recurrent, metastatic, or resistant cancer.

One embodiment of this aspect provides a pharmaceutical composition for treating recurrent, metastatic, and resistant cancers. The pharmaceutical composition according to the present disclosure can be administered to a subject suffering from cancer to treat cancer in the subject. One aspect of the present disclosure provides a method of treating cancer, comprising administering the pharmaceutical composition according to the present disclosure to a subject in need thereof. In this aspect, the subject in need thereof includes a subject in need of treating or preventing recurrent, metastatic, or resistant cancer. The subject in need may be, for example, a cancer patient after or before receiving cancer therapy. Other aspects of the present disclosure provide a method of preventing recurrence and/or metastasis of cancer, including administering a pharmaceutical composition according to the present disclosure to a subject in need thereof. The pharmaceutical composition according to the present aspect includes a substance binding to laeverin according to the present disclosure, and preferably the substance binding to laeverin is conjugated with a cytotoxic agent in the present disclosure.

The term "pharmaceutically acceptable carrier" refers to any ingredient other than the therapeutic agent according to the present disclosure and the ingredient is safe in subjects and has little allergic reactivity. The pharmaceutically acceptable carrier includes, for example, an aqueous or non-aqueous solvent suitable for pharmaceutical administration, a solution (e.g., saline), a cryoprotectant (e.g., glycerol), a water-soluble polymer (e.g., dextran), or a buffer (e.g., phosphate buffer).

The "subject" is, for example, a human or a non-human mammal. Non-human mammal is, for example, rodents such as a mouse, rats, guinea pigs, a hamsters; non-human primates such as chimpanzees; even-toed ungulate such as cattle, goat, sheep; odd-toed ungulate such as horses; pet animals such as rabbit, dog, cat. The subject is, for example, a human or a non-human primate (e.g., chimpanzee, monkey, gorilla, gibbon, and orangutan). The subject is preferably a human.

### (Method for assessing cancer recurrence, metastasis or resistance)

One aspect of the present disclosure provides a method comprising detecting a cancer cell expressing laeverin in a biological sample derived from a subject in need thereof by using a substance binding to laeverin and assessing recurrence, metastasis, or resistance of cancer-based on the detection result.

The "subject in need" is, for example, a person who was identified by an expert as suffering from cancer based on a particular diagnostic criteria. The subject in need may be, for example, a subject with stage I, II, III, or IV cancer according to a cancer stage classification (e.g., TNM classification provided by the International Union against Cancer (UICC)). The subject in need is, for example, a subject with stage II, III, or IV cancer. The subject in need is, for example, a subject with stage III or stage IV cancer.

A "biological sample" includes any tissue, tissue fragment, or body fluid taken from a subject. A biological sample is, for example, a tissue, tissue fragment, or bodily fluid that is expected to contain a cancer cell expressing laeverin. The biological sample may, for example, be the tissue, tissue fragment, or fluid itself taken from the subject and maybe a sample that has been preserved or de-purified. The biological sample is, for example, a portion of primary tumor tissue, a portion of metastatic tumor tissue, lymphoid tissue including lymph node, a tissue homogenate, and body fluid (e.g., blood, a fraction containing blood cell components, lymph fluid, pancreatic fluid, bile, ascites fluid). The biological sample is, for example, lymphatic fluid or lymphoid tissue. The biological sample is, for example, whole blood or a fraction containing peripheral blood mononuclear cells. The fraction containing peripheral blood mononuclear cells can be prepared, for example, by subjecting whole blood to density gradient centrifugation and specific gravity centrifugation (e.g., Ficoll-Paque^{®}, Lymphoprep^{®}). The biological sample is preferably a blood sample or lymph fluid, more preferably a blood sample.

The term "cancer cell(s) expressing laeverin" refers to a cancer cell(s) expressing a membrane-bound form of laeverin. A cancer cell expressing laeverin may be in the state of a single cancer cell or a cell aggregate (also referred to as "spheroid") containing multiple cancer cells expressing laeverin. The spheroid may, for example, contain cancer cells expressing laeverin and cancer cells not expressing laeverin. The spheroid may, for example, consist essentially of cancer cells expressing laeverin. The spheroid may, for example, consist of cancer cells expressing laeverin only.

Cancer cells expressing laeverin are capable of proliferating in a scaffold-independent manner. Cancer cells expressing laeverin can survive, for example, in the blood. Cancer cells expressing laeverin can survive, for example, in lymphatic vessels or lymph nodes. Cancer cells expressing laeverin can survive in the environment, for example, inside tumor tissue or surrounded by dead cells. Cancer cells expressing laeverin can proliferate in a scaffold-independent and scaffold-dependent manner. Cancer cells expressing laeverin preferably express IDO1. Cancer cells expressing laeverin are, for example, CTCs. Cancer cells expressing laeverin can be used as a material to identify a gene involved in cancer recurrence and/or metastasis by comparing gene expression patterns with cancer cells not expressing laeverin. Gene expression patterns can be analyzed, for example, by using a DNA microarray.

A substance binding to laeverin according to the aspect preferably includes a label substance. The term "label substance" refers to a substance that gives a capturable or detectable signal. The label substance may be, for example, a fluorescent dye, a magnetic particle, or a combination thereof. A capturable signal may be, for example, a magnetic signal. A detectable signal may be, for example, an optical signal.

The term "fluorescent substance" refers to a substance that absorbs excitation light and emits light (fluorescence) at a wavelength longer than the wavelength of the excitation light. Fluorescent substance may be, for example, fluorescein, rhodamine, Texas red, tetramethylrhodamine, carboxyrhodamine, phycoerythrin, 6-FAM^{™}, Alexa Fluor^{®}. The term "magnetic particles" includes particles composed of materials that do not have a magnetic field but form magnetic dipoles when exposed to a magnetic field. Magnetic particles have a particle size of, for example, about 1 nm to about 1 µm, about 1 nm to about 100 nm, and about 1 nm to about 10 nm. The magnetic particles comprise, for example, iron hydroxide, iron oxide hydrate, iron oxide, mixed iron oxide, or iron. The magnetic particles are, for example, magnetic particles with a particle size of 2 to 3 nm.

"Detect" includes an action of qualitatively or quantitatively finding a specific substance (e.g., a protein, nucleic acid, cell) and/or continuously monitoring the substance already found. Detection of cancer cells expressing laeverin in a biological sample includes, for example, mixing a substance binding to laeverin with the biological sample and counting the cancer cells to which the substances binding to laeverin were bound. Detection of cancer cells expressing laeverin in a biological sample includes, for example, mixing the biological sample with a substance binding to laeverin to which a label substance emitting a fluorescent signal is conjugated, subjecting the mixture to FACS, and counting the cancer cells to which the substances binding to laeverin were bound. The detection of cancer cells expressing laeverin in the assessment method is performed in vitro.

Assessment of recurrence, metastasis, or resistance includes, for example, assessing the cancer as being at risk of recurrence, metastasis, or resistance when the detection result indicates the presence of a cancer cell expressing laeverin in the biological sample. Assessment of cancer recurrence, metastasis, or resistance includes, for example, assessing that there is a risk of cancer recurrence and/or metastasis when the number of cancer cells expressing laeverin in the biological sample, as indicated by the detection result, is no less than a threshold value. The threshold is pre-determined based on various factors including, for example, the type of cancer, the age, weight, genetic background, and gender of the subject.

The assessment method according to the present disclosure can be performed automatically or semi-automatically based on the threshold value and the detection result or measurement of cancer cells expressing laeverin. Thus, a healthcare professional other than a physician (e.g., a medical informatics technician, a clinical engineer, or a clinical laboratory technician) can perform the assessment method in accordance with the present disclosure. The assessment method according to the present disclosure does not include, for example, a diagnostic act by a physician on a human being. The assessment method according to the present disclosure that does not include medical treatment by a physician on a human being corresponds to a method in which a healthcare professional other than a physician provides information for a physician to diagnose that the cancer is recurrent, metastatic, or resistant to cancer.

One aspect of the present disclosure provides a method for presenting information for diagnosing a subject with recurrent, metastatic, or resistant cancer. The method for the aspect includes detecting a cancer cell expressing laeverin in a biological sample from a subject in need thereof by using a substance binding to laeverin and presenting information for diagnosing or determining recurrent, metastatic, or resistant cancer based on the detection result.

In this aspect, a subject assessed as having resistant cancer may be administered a pharmaceutical composition according to the present disclosure after or during cancer therapy such as chemotherapy. In this aspect, a subject assessed as having recurrent and/or metastatic cancer may be diagnosed or assessed as having recurrent and/or metastatic cancer. Accordingly, one aspect of the present disclosure provides a method for diagnosing or determining whether a subject suffers from recurrent and/or metastatic cancer. The method according to the aspect includes detecting a cancer cell expressing laeverin in a biological sample from a subject in need thereof by using a substance binding to laeverin, and diagnosing or determining whether the subject is suffering from recurrent and/or metastatic cancer based on the detection result. The detection of the cancer cell expressing laeverin in the diagnostic or determining method is performed in vitro.

In one embodiment, when the biological sample is a blood sample (e.g., whole blood sample, plasma sample, or serum sample), the cancer cells expressing laeverin are blood-circulating tumor cells (CTCs). Thus, one aspect of the present disclosure provides a method for diagnosing or determining whether a subject has CTC. The method according to the aspect includes detecting a circulating tumor cell expressing laeverin in a blood sample (e.g., whole blood sample, plasma sample, or serum sample) from a subject in need thereof by using a substance binding to laeverin, and diagnosing or determining whether the subject has a CTC based on the detection result.

One aspect of the present disclosure provides a method for determining whether a cancer stem cell is present. The method according to the aspect includes detecting a cancer cell expressing laeverin in a biological sample from a subject in need thereof by using a substance binding to laeverin, and determining whether a cancer stem cell is present based on the detection result.

A subject assessed for recurrent, metastatic, or resistant cancer according to the aspect may be subjected to a method of preventing or treating recurrent, metastatic, or resistant cancer. Thus, one aspect of the present disclosure may comprise detecting a cancer cell expressing laeverin in a biological sample from a subject in need thereof by using a substance binding to laeverin; assessing or determining the subject as recurrent, metastatic, or resistant cancer based on the detection result; and administering to the subject assessed or determined as being recurrent, metastatic, or resistant cancer, a pharmaceutical composition according to the present disclosure or a pharmaceutical composition for treating cancer. The pharmaceutical composition for treating cancer may include, for example, a known anticancer agent, or a known immunotherapeutic agent, or a combination thereof. The pharmaceutical composition for treating cancer may contain a substance binding to laeverin.

### (Method for determining a substance impairing a cancer cell expressing laeverin)

One aspect of the present disclosure provides a method for determining a substance impairing a cancer cell expressing laeverin, comprising culturing a cancer cell expressing laeverin under a condition that allows contact with a test substance and determining the test substance as a substance impairing a cancer cell expressing laeverin based on the culture result.

A "test substance" is, for example, a small molecule compound, protein (e.g., antibody), DNA, RNA, small interfering RNA, or antisense oligonucleotide. The test substance binds, for example, to laeverin. The test substance preferably binds to laeverin with a predetermined binding capacity and possesses cytotoxic properties. The test substance is, for example, a cytotoxic agent-conjugated substance binding to laeverin. A cytotoxic agent-conjugated substance binding to laeverin is, for example, a substance binding to laeverin to which the cytotoxic agent directly binds.

Cancer cells expressing laeverin can be prepared, for example, from a cancer cell line or a biological sample. The biological sample is, for example, body fluid such as blood or lymphatic fluid from a cancer patient or tumor tissue or portion thereof. Cancer cell lines can be prepared, for example, from a biological sample according to known methods. Cancer cell lines are, for example, commercially available. Biological samples can be obtained from a subject using known methods.

Preparation of cancer cells expressing laeverin includes, for example, fractionating cancer cells expressing laeverin from a biological sample by using a substance binding to laeverin to which a labeling substance is conjugated (also referred to as a "laeverin detection reagent"). The preparation method includes, for example, mixing the biological sample and a laeverin detection reagent to allow a complex formation of the cancer cell and the laeverin detection reagent, and fractionating the complex based on a signal derived from the laeverin detection reagent in the complex. The fractionation of the complexes can be performed using, for example, a single cell/tissue picking device, FACS, MACS, or a combination thereof. The method for preparing cancer cells expressing laeverin includes, for example, removing at least one impurity in the biological sample to concentrate the desired cancer cells expressing laeverin. The cancer cells expressing laeverin may be, for example, CTCs, which may be prepared from a blood sample (e.g., whole blood sample, plasma sample, or serum sample) collected from a subject according to the above-described method for preparing cancer cells expressing laeverin from a biological sample or the preparation method described in Examples in the present disclosure.

Cancer cells expressing laeverin can be prepared, for example, by cell-suspension culture of the cancer cell line. Cell-suspension culture involves, for example, culturing a cancer cell line by shaking it in a low-absorbent culture plate. Flotation culture includes, for example, culturing a cancer cell line in a low-adsorption tube with rotating. Cell-suspension culture includes, for example, culturing them according to a hanging drop method. The flotation culture of a cancer cell line can be performed according to known cell culture conditions. Culturing cells according to a hanging drop method includes culturing them for 1 to 3 days, 1 to 2 days, or 1 day under known culture conditions.

Known cell culture conditions include, for example, maintaining cells at 37°C under 5% CO₂ Culture temperature is, for example, 37°C. A concentration of CO₂ is, for example, 5%. Cell suspension culture includes, for example, using a basic medium or medium supplemented with additives. The "basic medium" can be prepared according to known protocols or is commercially available. Basic medium is, for example, Dulbecco's Modified Eagle's Medium (DMEM), Minimum Essential Medium (MEM), or Basic Medium Eagle (BME). Additives include, for example, serum, growth factors, nutrients, or combinations thereof.

Culturing cancer cells expressing laeverin under a condition that allows contact with a test substance includes, for example, culturing the cells in a medium that is a basic medium supplemented with a test substance. The culture is, for example, an adhesion culture or a cell suspension culture. The culture is, for example, an adhesion culture. The culture is, for example, a cell suspension culture.

Determining a test substance as a substance impairing a cancer cell expressing laeverin includes determining the test substance as a substance that inhibits or arrests cancer cell growth or kills or destroys cancer cells if the culture result indicates as described below.

If the number of cancer cells expressing laeverin is the same before and after culture with the test substance, it can be determined as a substance capable of arresting the growth of the cancer cells expressing laeverin. If the number of cancer cells expressing laeverin is reduced after culture compared to before culture, the test substance can be determined as a substance capable of killing or destroying the cancer cells expressing laeverin. If the growth rate of the cancer cells expressing laeverin cultured in the medium without the test substance (negative control) is smaller than the growth rate of the cancer cells expressing laeverin cultured in the medium to which the test substance is added, the test substance can be determined as a substance capable of inhibiting the growth of the cancer cells expressing laeverin. The proliferation rate is defined as the rate of proliferation of the number of cancer cells before culture. The proliferation rate is the ratio of the number of cells after culture to the number of cells before culture.

### (Method for determining a factor influencing cancer recurrence, metastasis, or resistance)

One aspect of the present disclosure provides a method for determining a factor influencing cancer recurrence, metastasis, or resistance, comprising comparing a cancer cell expressing laeverin with a cancer cell that does not express laeverin but has the same origin as the cancer cell expressing laeverin to determine a difference in the genetic or proteinaceous factor.

Cancer cells expressing laeverin can be prepared, for example, from a cancer cell line or a biological sample. Cancer cells expressing laeverin can be prepared, for example, by fractionating cancer cells expressing laeverin from a biological sample (tumor tissue or tumor tissue fragments) by using a reagent for detecting laeverin. Cancer cells expressing laeverin can be prepared, for example, by culturing a cancer cell line in suspension.

The term "cancer cell that does not express laeverin" refers to a cancer cell that expresses less than twice the amount of laeverin in an adhesion cultured cancer cell line. The expression level of laeverin in this context is the amount of mRNA encoding laeverin. The amount of mRNA encoding laeverin is measured by RT-qPCR. The amount of mRNA encoding laeverin is normalized against mRNA encoding, for example, actin. Cancer cells that do not express laeverin are cancer cells that express less than twice the amount of laeverin in, for example, chorionic ectoderm (EVT)-derived Swan71 cells. Cancer cells that do not express laeverin, for example, may be in a single cell state or in a cell aggregate (e.g., spheroids).

Cancer cells that do not express laeverin can be prepared, for example, from cancer cell lines or biological samples. The preparation method can be performed, for example, by adhesion culture of the cancer cell line. The preparation method can include, for example, mixing a biological sample and a laeverin detection reagent to form a complex of the cancer cell and the laeverin detection reagent, and fractionating the cancer cells based on the absence of detection of a signal derived from the laeverin detection reagent from the cancer cells. The preparation method may further include fractionating the complex based on detection of a signal derived from the laeverin detection reagent in the complex. The fractionation of the complexes and the fractionation of cancer cells to which the laeverin detection reagent is not bound, i.e., cancer cells that do not express laeverin, can each be performed using, for example, a single cell/tissue picking device, FACS, or MACS or a combination thereof.

In this aspect, the cancer cells that do not express laeverin are cancer cells that are of the same origin as the cancer cells expressing laeverin. In this context, the term "same origin" means that the cancer cells expressing and not expressing laeverin originate from the same animal species and from the same primary tumor tissue. The same animal species may be, for example, the same individual or different individuals. Cancer cells derived from the same primary tumor tissue may, for example, both cancer cells not expressing and cancer cells expressing laeverin may be derived from the same primary tumor tissue, or one may be derived from the primary tumor tissue and the other from metastatic tumor tissue.

Genetic factors include, for example, mutations in gene sequences, the presence or absence or extent of transcription, and chemical modifications such as DNA methylation. Proteinaceous factors include, for example, chemical modifications such as methylation and ubiquitination of histones, the presence or absence or extent of expression, and post-translational modifications such as the addition of sugar chains.

The determination of a difference in genetic factors or proteinaceous factor does not preclude the determination of both genetic factor difference and proteinaceous factor difference. Identification of a difference in genetic factor or proteinaceous factor can be done, for example, by comparing the result of genetic factor measurement or proteinaceous factor measurement. The measurement result may be, for example, electronic data. One aspect of the present disclosure includes comparing measurement results for a genetic factor or a proteinaceous factor in a cancer cell expressing laeverin with measurement results for the factor in a cancer cell that does not express laeverin but has the same origin as the cancer cell, and determining differences in the genetic factor or the proteinaceous factor. The method of determining a factor that influences cancer recurrence, metastasis, or resistance is provided, including determining the difference.

### (Use of laeverin as a biomarker for a cancer stem cell)

One aspect of the present disclosure provides a use of laeverin as a biomarker for a cancer stem cell. Another aspect of the present disclosure provides a biomarker for a cancer stem cell, the biomarker consisting of laeverin.

Examples in the present disclosure demonstrate that cancer cells surrounded by necrotic cancer cells inside primary tumor tissue express laeverin and survive. It is suggested that these cancer cells expressing laeverin can survive by suppressing their cell proliferation rate, etc., in an environment where cancer cells surrounding them will die. Examples in the present disclosure also demonstrate that the cancer cells expressing laeverin can enter lymphatic vessels and blood vessels, evade immune system attacks, circulate in the body, and metastasize to organs other than the primary tumor tissue (e.g., lymph nodes). Further, Examples in the present disclosure demonstrate that cancer cells in metastatic tissues do not or little express laeverin and that cancer cells detached from the metastatic tissue express laeverin at a high level. In addition, Examples in the present disclosure demonstrate that cancer cells resistant to chemotherapeutic agents express laeverin. These characteristics are similar to those of cancer stem cells, which are thought to be attributed to cancer recurrence and/or metastasis. Thus, laeverin can be used as a biomarker for a cancer stem cell.

As described above, Examples in the present disclosure demonstrate that cancer cells expressing laeverin can enter lymphatic vessels or blood vessels and metastasize to organs other than the primary tumor tissue. The characteristics correspond to a mechanism of cancer recurrence and/or metastasis. Therefore, laeverin can be used as a biomarker for cancer recurrence and/or metastasis. One aspect of the present disclosure provides a use of laeverin as a biomarker of cancer recurrence and/or metastasis. Another aspect of the present disclosure provides a biomarker for cancer recurrence and/or metastasis, the biomarker consisting of laeverin.

The term "cancer stem cell" refers to a cancer cell with a quiescent phase. The term "quiescent phase" refers to a period during which the viable cell density is nearly constant (i.e., within a measurement error). For example, cancer stem cells in quiescence show a nearly constant viable cell density for at least 24 hours (e.g., 36 hours or more). Cancer stem cells can divide asymmetrically when they leave the quiescent phase and are in the proliferative phase. The term "asymmetric division" refers to cell division that gives rise to different daughter cells. Asymmetric division of a cancer stem cell can give rise to a cancer stem cell and a cancer cell differentiated from the cancer stem cell. The term "proliferative phase" refers to increased viable cell density. The proliferative phase can be divided into the DNA synthesis phase (S phase), the mitotic phase (M phase), and the Gap phase (G1 and G2 phases) between these phases according to morphological or biochemical measurements.

The term "biomarker" refers to an in vivo substance, such as a peptide (including protein) or nucleic acid (including gene or its transcript) present in a cell, tissue, or body fluid, which can be an indicator of the presence, change, or progress of a disease or degree of therapeutic effect according to its presence or concentration. A biomarker according to the present disclosure is laeverin. Laeverin is used, for example, as a biomarker for metastatic, recurrent, or resistant cancer. For example, Laeverin is used as a cancer stem cell biomarker.

The use of laeverin as a biomarker includes detecting the presence of a cancer cell expressing laeverin in a biological sample from a subject in need thereof, e.g., to detect whether a cancer stem cell is present. The use of laeverin as a biomarker includes detecting the presence of a cancer cell expressing laeverin in a biological sample from the subject to determine whether the subject has recurrent, metastatic, or resistant cancer. A cancer cell expressing laeverin can be detected and/or measured according to known immunological methods, particularly the methods described in the present disclosure (e.g., as described in the Examples).

The term "comprising" herein means inclusive of recited elements and/or steps and other additional elements and/or steps. The term "consisting of" herein means inclusive of recited elements and/or steps but exclusive of other additional elements and/or steps. The term "consisting essentially of" herein means inclusive recited elements and/or steps and inclusive other additional elements and/or steps to the extent that they do not affect the novel technical features of the pharmaceutical compositions and methods. The term "not substantially comprising" herein does not exclude "completely excluding.

Unless described otherwise, the terms and explanations for embodiments provided by the present disclosure appropriately apply to other aspects and embodiments provided by the present disclosure.

Specific examples are described below, but they represent preferred embodiments of the present disclosure and are not intended to limit in any way the invention as recited in the Claims.

### EXAMPLES

### [EXAMPLE 1] laeverin expression in spheroids formed from cancer cells (Materials)

MCF7, SKBR3, and BT20 cell lines were used as mammary carcinoma cells. A375 and SK-MEL28 cell lines were used as melanoma cells. HEC6 and HEC108 cell lines were used as uterine cancer cells. SKOV3 cell line was used as ovarian cancer cells.

### (Adhesion culture)

Cancer cells were pre-cultured in culture dishes for adherent cells at 37 °C. Cultured cells were collected when they reached 90-100% confluence. Collected cultured cells were seeded at 1 × 10⁵ cells per well and incubated at 37 °C for 24 hours.

### (Cell-suspension culture)

The pre-cultured cancer cells were seeded on culture dishes for floating cells or on ultra-low adhesion culture plates at 1 to 2 x 10⁶ cells per well and incubated at 37 °C to form spheroids. The cultured cells were collected and transferred to flasks, which were incubated on a wave shaker at 37 °C for 24-72 hours.

### (Fluorescence immunostaining method)

Cells grown in adhesive or cell-suspension culture were fixed using 4% paraformaldehyde. Laeverin expression in fixed cells was examined using an anti-laeverin antibody (specifically, monoclonal 5-23 antibody (10 µg/ml) or anti-LVRN antibody was used) and a sheep anti-mouse IgG antibody conjugated with Alexa488. Hoechst 33342 was used for nuclear staining. The cancer cell lines were each grown in adhesion culture (Comparison Examples 1 to 8). As a positive control, extravillous trophoblast (EVT)-derived Swan71 cells overexpressing laeverin, Swan71 LVRN, were used.

MCF7 cells grown in adhesion culture (Comparison Ex. 1) did not express laeverin (FIG. 1a). The positive control cells grown in adhesion culture expressed laeverin (FIG. 1d). Similar tests were conducted with other cell lines (Comparative Ex. 2 to 8). The other cell lines grown in adhesion culture did not express laeverin. The 5-23 antibody was used for immunostaining for laeverin. These results are summarized in the table below.

**[Table 1]**

| | Cell line | Cultured cells form | Laeverin expression |
|---|---|---|---|
| Comparison Ex. 1 | MCF7 | adherent-cultured cells | NO |
| Comparison Ex. 2 | SKBR3 | adherent-cultured cells | NO |
| Comparison Ex. 3 | BT20 | adherent-cultured cells | NO |
| Comparison Ex. 4 | A375 | adherent-cultured cells | NO |
| Comparison Ex. 5 | SK-MEL28 | adherent-cultured cells | NO |
| Comparison Ex. 6 | HEC6 | adherent-cultured cells | NO |
| Comparison Ex. 7 | HEC108 | adherent-cultured cells | NO |
| Comparison Ex. 8 | SKOV3 | adherent-cultured cells | NO |
| Test Ex. 1 | MCF7 | spheroids | YES |
| Test Ex. 2 | SKBR3 | spheroids | YES |
| Test Ex. 3 | BT20 | spheroids | YES |
| Test Ex. 4 | A375 | spheroids | YES |
| Test Ex. 5 | SK-MEL28 | spheroids | YES |
| Test Ex. 6 | HEC6 | spheroids | YES |
| Test Ex. 7 | HEC108 | spheroids | YES |
| Test Ex. 8 | SKOV3 | spheroids | YES |

| | | | |
|---|---|---|---|
| NO: No expression of laeverin could be observed. YES: Expression of laeverin was observed. | | | |

The cancer cell lines were each grown in cell-suspension culture to form spheroids (Test Examples 1 to 8). MCF7 cell spheroids (Test Ex. 1) expressed laeverin (FIG. 2a and FIG. 2c). Similar tests were conducted with other cell lines (Test Ex. 2 to 8). Every cell line's spheroids formed in cell-suspension culture expressed laeverin. Immunostaining for laeverin was performed using the 5-23 or anti-LVRN antibody. These results are summarized in the table above.

Example 1 indicates that cancer cells do not express laeverin when grown in a scaffold-dependent manner by adhesion culture but express laeverin when grown in a scaffold-independent manner by cell-suspension culture. Example 1 reveals that cancer cell spheroids (cell clusters) express laeverin.

### [EXAMPLE 2-1] Expression level of laeverin in cancer cell spheroids

In a similar way to Example 1, the cultured cell lines were each grown in adhesion culture or cell-suspension culture (shaking culture for 72 hours). The expression level of laeverin in cultured cancer cells was examined (FIG. 3). In A375 (FIGs.3a-d) and A2780 (FIGs. 3i-l) cell lines grown in a scaffold-dependent manner by adhesion culture, laeverin expression was not observed (FIG. 3b and FIG. 3j). In the cancer cell spheroids grown in a scaffold-independent manner by cell-suspension culture (FIGs. 3e-h and m-p), laeverin expression was observed (FIGs. 3f and 3n). These results were the same as those in Example 1.

### (RT-qPCR)

The A375 and A2780 cell lines were each grown in adhesion culture. The cultured cells were collected, and total RNA was recovered from each. In a similar way, the cell lines were each grown in cell-suspension culture with a wave shaker to form spheroids. The spheroids were collected, and total RNA was recovered from each. The amount of mRNA encoding laeverin was measured by RT-qPCR with each recovered total RNA. The amount of the mRNA reflects the level of the laeverin expression. The RT-qPCR used a reverse transcriptase and a primer set, allowing the amplification of the laeverin sequence. The laeverin expression level of the A375 cells was about 14-fold higher in the cell-suspension culture than in the adhesion culture (FIG. 4a). Similarly, the laeverin expression level of the A2780 cells was about 8-fold higher in the cell-suspension culture than in the adhesion culture (FIG. 4b).

CaSki and SiHa cell lines are cervical cancer cell lines infected with human papillomavirus 16 (HPV16), respectively. The cell lines were each grown in adhesion culture, and the cultured cells were collected. The cell lines were each grown in cell-suspension culture (on ultra-low adhesion culture plates in shaking culture for 24 hours) to form spheroids. The spheroids were collected. Total RNA was recovered from each of the collected cells grown in adhesion culture and the collected cells (spheroids) grown in cell-suspension culture. The total RNAs were used to examine the laeverin expression levels. The results indicate that the laeverin expression level was about 3.5-fold higher in the cell spheroids than in the adhesion-cultured cells (FIG. 4c and 4d).

Examples 1 and 2 reveal that even cancer cells expressing no laeverin come to express laeverin or enhance the expression when grown in a scaffold-independent manner by cell-suspension culture.

### [EXAMPLE 2-2.]

### (Materials)

The A375 cell line was used as melanoma cells. The HCT116 cell line was used as colon cancer cells. The HCC38 cell line was used as breast cancer cells. The LNCap, DU145, and PC-3 cell lines were used as prostate cancer cells.

### (Spheroid formation)

Pre-cultured cancer cells were seeded in ultra-low adhesion culture plates at 1 × 10⁵ cells per well and cultured at 37 °C for 3-4 days to form spheroids. The cultured cells were collected and transferred to flasks, which were incubated on a wave shaker at 37 °C for 7 days.

### (Fluorescence immunostaining method)

The formed spheroids were subjected to fluorescent immunostaining in a similar way to Example 1 to test whether the spheroids expressed laeverin. The 5-23 antibody was used for the laeverin immunostaining.

Example 2-2 demonstrates that spheroid-formed melanoma cells (A375), colon cancer cells (HCT116), breast cancer cells (HCC38), and prostate cancer cells (LNCap, DU145, and PC-3) express laeverin. These results are summarized in the table below.

**[Table 2]**

| | Cell line | Cultured cells form | Laeverin expression |
|---|---|---|---|
| Test Ex. 9 | A375 | spheroids | YES |
| Test Ex. 10 | HCT116. | spheroids | YES |
| Test Ex. 11 | HCC38 | spheroids | YES |
| Test Ex. 12 | LNCap | spheroids | YES |
| Test Ex. 13 | DU145 | spheroids | YES |
| Test Ex. 14 | PC-3 | spheroids | YES |

| | | | |
|---|---|---|---|
| NO: No expression of laeverin could be observed. YES: Expression of laeverin was observed. | | | |

### (RT-qPCR)

The laeverin expression level in the cancer cells of spheroids grown in cell-suspension culture was measured by RT-qPCR in a similar way to Example 2. The laeverin expression level in the cancer cells of monolayers grown in adhesion culture was also measured by RT-qPCR. Each expression level was normalized against the expression level of Hprt1 (FIG. 20).

Figure 20 shows that the cancer cells used in Example 2-1 come to express laeverin by forming spheroids. Specifically, the melanoma cell line A375 had approximately 79-fold higher levels of laeverin expression in spheroids than in monolayers. Similarly, the colon cancer cell line HCT116 had approximately 33-fold higher expression levels in spheroids. The breast cancer cell line HCC38 had approximately 37-fold higher expression levels in spheroids. The prostate cancer cell lines LNCap, DU145, and PC-3 had approximately 7-, 11-, and 13-fold higher expression levels in spheroids, respectively.

Examples 1 and 2-2 reveal that cells derived from various cancers (e.g., mammary, melanoma, uterine, ovarian, colon, breast, and prostate) come to express laeverin at a high level by forming spheroids.

### [EXAMPLE 3] laeverin expression in circulating tumor cells (CTCs) in the blood (Isolation of CTCs)

Fractions containing peripheral blood mononuclear cells (PBMCs) were recovered from peripheral blood samples derived from subjects. PBMCs expressing CD45 were removed from the fractions to prepare blood samples enriched for circulating tumor cells (CTCs). CTCs co-expressing laeverin and EpCAM were identified from the blood samples. The identified CTCs were isolated and prepared.

More specifically, the following proceedings were carried out to isolate the CTCs.

Peripheral blood mononuclear cells (PBMCs) were prepared from the whole blood by collecting 7 ml of peripheral blood from the subjects and subjecting the collected whole blood to density gradient centrifugation and specific gravity centrifugation using Ficoll-Paque^{®}. The PBMCs were mixed with a mouse monoclonal anti-LVRN antibody (10 µg/ml) and a rabbit polyclonal anti-EpCAM antibody (10 µg/ml), and the resulting mixture was incubated at room temperature for 30 minutes. The mixture was mixed with an Alexa488-conjugated goat anti-mouse IgG antibody (1:500 dilution) and a CF555-conjugated goat anti-rabbit IgG antibody (1:500), and the mixture was incubated at room temperature for 30 minutes. The mixture was mixed with magnetic beads immobilized with an anti-CD45 antibody, and the resulting mixture was incubated at 4 °C for 15 minutes. After the incubation, the mixture was mixed with a BV421-conjugated anti-CD45 antibody, and the resulting mixture was further incubated at 4 °C for another 30 minutes.

After the incubation, the mixture was subjected to a magnetic cell separation system (MACS) to remove CD45-positive cells, resulting in enriched CD45-negative cells. The enriched cells were injected into each well of a 96-well cell imaging plate and observed under a fluorescence microscope. When fulfilling CTC criteria, cells were identified as blood-circulating tumor cells (CTCs). The CTC criteria used were LVRN-positive and/or EpCAM-positive and CD45-negative. Culture mediums in wells that indicate the presence of CTCs were collected as the inclusion of CTCs and dropped on 35 mm culture dishes to form droplets. Cells fulfilling the CTC criteria were isolated and recovered from the cell population in the droplets with a Unipick^{®} single cell and tissue-picking device.

### (Specimens A and B)

Patient A suffered from stage IIIB cervical cancer with multiple lymph node metastases. The patient's cancer recurred after concurrent chemo-radiation therapy (CCRT). After the recurrence, the patient was treated with a combination therapy of paclitaxel and carboplatin (TC therapy), followed by a combination therapy of cisplatin and vinorelbine (NP therapy), and then irinotecan (CPT-11). After discontinuing the CPT-11 therapy, a peripheral blood sample was collected from Patient A (Specimen A).

Patient B suffered from stage IIIC cancer of the uterine body with multiple brain, skin, and lymph node metastases. The patient underwent abdominal total hysterectomy (ATH), bilateral sarcoma removal (BSO), omentectomy (OMT), pelvic lymph node dissection (PLAN), and para-aortic lymph node dissection (PAN) before the patient's cancer recurred. The patient received TC therapy, followed by a combination therapy of doxorubicin and cisplatin anticancer drug (AP therapy). While undergoing best supportive care (BSC) after the AP therapy, a peripheral blood sample was collected from Patient B (Specimen B).

CTCs in specimens A and B were identified according to the method for identifying circulating tumor cells in the blood described above. In Figure 5, the white arrows show cell aggregates expressing laeverin (FIG. 5a) and EpCAM (FIG. 5b) but not express CD45 (FIG. 5c). Figure 5 indicates that the cell aggregates are CTCs expressing laeverin.

CTCs in specimens A and B were assessed according to the above CTC criteria. As a result, 8 CTCs were identified in Specimen A, and 15 CTCs were identified in Specimen B. Two of the 8 CTCs in Specimen A formed cell aggregates, and six were single cells. Of the 8 CTCs in Specimen A, one CTC was positive only for EpCAM, one CTC was positive only for LVRN, and 6 CTCs were positive for both EpCAM and LVRN. Two of the 15 CTCs in Specimen B formed cell aggregates, and 13 were single cells. Four CTCs of the 15 CTCs in Specimen B were positive only for EpCAM, two CTCs were positive only for LVRN, and 9 CTCs were positive for both EpCAM and LVRN. In Specimen B, CTC cell aggregates containing leukocytes positive for CD45 were observed. These results are summarized in the table below.

**[Table 3]**

| Specimen | The number of CTCs | The number of cell aggregates | The number of single cells | LVRN⁻ & EpCAM⁺ | LVRN⁺ & EpCAM⁻ | LVRN⁺ & EpCAM⁺ |
|---|---|---|---|---|---|---|
| A | 8 | 2 | 6 | 1 | 1 | 6 |
| B | 15 | 2 | 13 | 4 | 2 | 9 |

### [EXAMPLE 4] laeverin expression in CTCs in additional biological specimens (Specimen C)

Patient C suffered from stage IIIB cervical cancer with liver metastases and multiple lymph node metastases. After the cervical cancer developed stage IIIB, a peripheral blood sample was collected from Patient C (specimen C).

### (Improved method for CTC isolation)

CTCs were isolated from specimen C using a CTC preparation method improved from that of Example 3. The CTC isolation method of Example 3 involves excluding PBMCs expressing CD45 to enrich CTCs, while the improved method involves collecting cancer cells expressing EpCAM to enrich CTCs.

CTCs from specimen C were isolated using an improved method, including the following proceedings.

Peripheral blood was collected from a subject, and peripheral blood mononuclear cells were recovered from the collected peripheral blood. The collected peripheral blood mononuclear cells were fixed with 2% paraformaldehyde. A sample containing the fixed cells was mixed with mouse monoclonal anti-LVRN antibody (10 µg/ml), Alexa488 conjugated anti-mouse IgG antibody (1:500 dilution), and BV421 conjugated anti-CD45 antibody. The resulting mixture was incubated at 4 °C for 30 minutes. The mixture was mixed with anti-EpCAM antibody bound to magnetic beads, and the resulting mixture was incubated at 4 °C for 30 minutes. The mixture was subjected to a magnetic cell separation system (MACS) to enrich EpCAM-positive cells. A culture medium of 10 µL containing the concentrated cells was dropped onto a glass bottom dish coated with fluid paraffin to form 14 droplets. Cells in the droplets were observed under a fluorescence microscope, and cells that were LVRN positive and CD45 negative were assessed as blood circulating tumor cells (CTCs). CTCs were isolated and collected from the droplets, which showed the presence of CTCs, with a Unipick^{®} single cell and tissue picking device.

Five CTCs (Laeverin positive and CD45 negative) were separated from Sample C. Three of the five CTCs were isolated and collected; the three CTCs were mixed, and the total genomic DNA was amplified using a Single Cell Whole Genome Amplification Kit. DNA fragments were amplified using the amplified genomic DNA and a primer set that amplifies HPV16 (E6/E7). The strand length (131 bp) of the DNA amplification product amplified from the isolated CTCs was identical to that (131 bp) of the DNA product amplified from the peripheral blood sample before the CTC isolation. Both amplification products were generated using the same primer set. The result indicates that the CTCs expressing laeverin are cancer cells infected with HPV16 and that the improved method of CTC isolation was able to isolate CTCs of interest.

### (Another improved method)

In the improved method described above, after immobilizing cells with 2% paraformaldehyde, cells expressing laeverin and not expressing CD45 were isolated using anti-LVRN and anti-CD45 antibodies. In another improved method, after fixation of cells with 2% paraformaldehyde, in addition to the anti-LVRN and anti-CD45 antibodies, Hoechst is used to isolate cells that express laeverin and do not express CD45.

In the further improved method, a fraction containing peripheral blood mononuclear cells (PBMCs) was collected from a peripheral blood sample derived from a subject. Cells in the fraction were fixed using 2% paraformaldehyde. EpCAM-expressing cells were collected from the fixed cells to prepare a CTC-enriched blood sample. From the blood sample, CTCs expressing laeverin and no CD45 and being stained with Hoechst were detected. The detected CTCs were isolated to prepare CTCs.

### (Specimen D)

Patient D suffered from stage IVB cervical cancer with multiple lymph node metastases (bone marrow, para-aortic, and supraclavicular lymph nodes), left lower extremity DVT, external jugular vein thrombus, and left pancreatopathy (ureteral stent placement). Cancerous tissue fragments taken from Patient D indicated HPV type 16 positive squamous cell carcinoma. Patient D received therapy with a combination of cisplatin and paclitaxel anticancer agents (TP therapy), followed by Keytruda^{®} (pembrolizumab). A peripheral blood sample (Specimen D) was collected from Patient D after undergoing Keytruda.

The further improved method detected 56 CD45-negative CTCs from Specimen D of 1 ml. The result indicates that Specimen D was very rich in CTCs. Of the 56 CTCs, 3 cells expressed laeverin. LVRN-positive CTCs were mixed. The total genomic DNA was amplified from the resulting mixture using a Single Cell Whole Genome Amplification Kit (genomic DNA-p). Five laeverin-negative CTCs were mixed to prepare a mixture containing LVRN-negative CTCs. Two more mixtures containing LVRN-negative CTCs were prepared. For each of the three mixtures containing LVRN-negative CTCs, total genomic DNAs were amplified as described above (genomic DNA-n1, -n2, and -n3). The amplified genomic DNAs (genomic DNA-p, -n1, -n2, and -n3) were amplified with the primer set for amplifying HPV16 (E6/E7) to obtain DNA fragments.

The strand length (131 bp) of the DNA amplification products amplified from the total genomic DNAs (genomic DNA-p, -n1, -n2. and -n3) extracted from the CTC mixtures after the isolation was identical to that of the DNA amplification product amplified from peripheral blood samples before the CTC isolation (131 bp). Both amplification products were generated using the same primer set. The result indicates that CTCs isolated by the further improved method for CTC isolation are HPV16-infected cancer cells regardless of the expression of laeverin.

PBMCs expressing CD45 were excluded to enrich CTCs in the CTC isolation method of Example 3. The CTC isolation method in Example 3 detected 8 cells as CTC in Specimen A, and the number of CTCs expressed EpCAM only was one (12.5%) of them. In Specimen B, 15 cells were detected as CTC, and the number of CTCs expressed EpCAM only was 4 CTCs (about 27%) of them. The further improved method in Example 4 detected 56 cells as CTC, and the number of CTCs expressed EpCAM only was 53 (about 95%) of them. The improved and further improved methods collect cells expressing EpCAM to enrich CTCs. These results suggest that the CTC isolation method in Example 3 may have lost EpCAM-positive CTCs. In addition, these results suggest that enriching CTCs by isolating cells expressing laeverin leads to a more effective collection of CTCs expressing laeverin.

Examples 3 and 4 demonstrate that a single cancer cell or cell aggregate circulating in the blood expresses laeverin.

CTCs not expressing laeverin accounted for 1 out of 8 CTCs (12.5%) in Specimen A, 4 out of 15 CTCs (about 27%) in Specimen B, and 53 out of 56 (about 95%) in Specimen D. Subjects A, B, and D all suffered from metastatic cancer. Subject A had stage IIIB cervical cancer, Subject B had stage IIIC uterine cancer, and Subject D had stage IVB cervical cancer. These results indicate that cancer cells, even those that do not express laeverin, can circulate in the blood in later-stage cancers. On the other hand, in earlier-stage cancers, it is suggested that cancer cells expressing laeverin can circulate in the blood, but those not expressing laeverin may have difficulty doing so.

### [EXAMPLE 5] Effect of laeverin on the immune cell system.

The CaSki cell line was cultured in ultra-low-attachment culture plates for 24 hours with shaking, as described in Example 1, to form floating cancer cell spheroids. Laeverin and indoleamine 2,3-dioxygenase-1 (IDO1) mRNA levels were measured using RT-qPCR (FIG. 6). The expression level of laeverin (LVRN) in CaSki cells grown in cell suspension culture was about 4-fold higher than when they were grown in adhesion culture (FIG. 6, LVRN on the left side), consistent with the results in Example 2 (FIG. 4c). The expression level of IDO1 in the CaSki cells grown in cell suspension culture was about 23-fold higher than when they were grown in adhesion culture (FIG. 6, IDO1 on the right side). These results suggest that IDO1 expression is upregulated in cancer cell spheroids expressing laeverin

The expression of IDa 1 in tumor cells can lead to the differentiation to regulatory T cells (Tregs) under the area around the tumor, and this can promote the survival of cancer cells (Liu et al., Targeting the IDO1 pathway in cancer: from bench to bedside. Journal of Hematology & Oncology (2018)11: 100). IDO1 breaks down tryptophan, an essential nutrient for T cell function and survival. Tumor cells with high levels of IDO1 degrade tryptophan in their vicinity, leading to suppression of T cell activity around the tumor cells. In light of these, Example 5 suggests that cancer cells expressing laeverin or spheroids containing the cancer cells may induce Treg differentiation and evade attack the immune system's attacks due to the high expression of IDO1.

Examples 3-5 suggest that cancer cells expressing laeverin expressed IDOl, allowing them to evade the immune system's attacks in the blood, thereby circulating in the blood and proliferating at different sites than the primary tumor tissue. This indicates that cancer cells expressing laeverin play an essential role in cancer recurrence and/or metastasis.

### [EXAMPLE 6] Effect of laeverin-expressing cells on monocyte-derived cells

Transfectants of Swan71 cells overexpressing laeverin (Swan71_LVRN) were co-cultured with the human monocyte-derived cell line THP-1. Co-culturing was conducted under a condition that allowed contact between Swan71_LVRN cells and THP-1 cells. Cultured THP-1 cells were collected, and mRNA was extracted. The mRNA was subjected to a microarray analysis. As described above, THP-1 cells were co-cultured with Swan71 cells (Control) and THP-1, and the microarray analysis was performed with the cultured THP-1. The expression levels of several genes were increased in THP-1 cells co-cultured with Swan71_LVRN compared to THP-1 cells co-cultured with the Control.

The expression levels of four of the multiple genes described above were examined by RT-qPCR (FIG. 7). The four genes were 2'-5'-Oligoadenylate Synthetase 2 (OAS2), Interferon Induced Protein With Tetratricopeptide Repeats 1(IFIT1), IFIT3, and interferon-stimulated gene, 15kDa (ISG15).

Swan71_LVRN cells were co-cultured under a condition that allowed contact with THP-1 cells. Cultured THP-1 cells were collected, and mRNA was extracted from them. The extracted mRNA was subjected to RT-qPCR to examine the expression levels of each gene. The expression levels of the four genes examined were significantly increased (FIG. 7a). The expression levels of the four genes were measured as described above, except that the Swan71_LVRN cells were cultured under a condition that did not allow contact between them and THP-1 cells. In the culture condition, both cell types were present in each fraction, and the liquid components, including exosomes, were permeable between their fractions. No significant differences existed in the expression levels of the four genes examined (FIG. 7b).

Example 6 indicates that contact with cells expressing laeverin increases the expression level of the specific genes in monocytic cells.

### [EXAMPLE 7] Effect of soluble laeverin on monocyte-derived cells

There are two types of laeverin: membrane-bound laeverin and secreted laeverin. Recombinant laeverin (rLVRN) was constructed by combining the secreted form of laeverin with a His tag. THP-1 cells were grown in a culture medium that contained rLVRN. The expression levels of the four genes in the cultured THP-1 cells were measured. The results showed that the expression levels of the genes measured increased with increasing amounts of rLVRN added (FIG. 8a). The expression levels of the genes increased with longer culture time in the presence of rLVRN (4, 8, and 12 hours).

THP-1 cells were cultured with fluorescent dye-labeled rLVRN and observed under a microscope. As a result, the observation revealed the presence of rLVRN in the THP-1 cells, indicating that monocytes uptake laeverin.

Beads capable of binding to His tags are mixed with rLVRN to prepare beads immobilized with rLVRN. THP-1 cells were cultured in the presence of the rLVRN-immobilized beads or rLVRN in free form. Expression levels of the four genes mentioned above in the cultured THP-1 cells were measured. The levels of the four genes' expressions induced by rLVRN-immobilized beads (◆) were significantly suppressed in comparison with the levels of the four genes' expressions induced by rLVRN in free form (■) (FIG. 8b). The results suggest that the induction of these genes' expression by laeverin in monocytic cells is not sufficient by contact between monocytic cells and laeverin alone, but requires that laeverin be taken up into the cells.

The results of Examples 6 and 7 show that monocytic cells express certain genes at higher levels by taking up laeverin.

### [Example 8] Effect of soluble laeverin on human peripheral blood mononuclear cells

Human peripheral blood mononuclear cells (PBMCs), which include lymphocytes and monocytes, were cultured in the presence of rLVRN, and the expression levels of four genes, OAS2, IFIT1, IFIT3, and ISG15, were measured. Similar to the results with THP-1 cells, the expression of the four genes was induced.

Examined was which types of cells in peripheral blood mononuclear cells (PBMCs) were induced by laeverin to express ISG15.

EDTA-treated blood 3 ml was processed with Ficoll to prepare PBMCs. The PBMCs were cultured for 24 hours in a Floating Cells Dish containing 2 ml of RPMI medium. After that, rLVRN 1.5 µg/ml, INF-β 1000 IU/ml, or PBS (as a negative control) was added to the medium. After an additional 24 hours of culture, ISG15 in the cells was stained with phycoerythrin (PE). The PE-stained PBMCs were sorted into monocyte and lymphocyte fractions using the BD FACS Aria^{®} Fusion cell sorter.

The measurements with the monocyte fraction showed that ISG15 was expressed in 13.4% of monocytes cultured with PBS, 95.0% of monocytes cultured with rLVRN, and 95.4% of monocytes cultured with INFβ, respectively. In the lymphocyte fraction, ISG15 was expressed in 29.4%, 91.8%, and 92.6% of lymphocytes cultured with PBS, rLVRN, and INFβ, respectively. These results indicate that laeverin induces ISG15 expression in both monocyte and lymphocyte fractions, and there is no difference between laeverin and INFβ in inducing ISG15 expression in monocyte and lymphocyte fractions.

Examined was whether any of B cells, T cells, and CD14-positive monocytes were induced by laeverin to express ISG15.

EDTA-treated blood 3 ml was processed with Ficoll to prepare PBMCs. The PBMCs were cultured for 12 hours in a Floating Cells Dish containing 2 ml of RPMI medium. After that, rLVRN 1.5 µg/ml or PBS (as a negative control) was added to the medium. After an additional 24 hours of culture, ISG15 in the cells was stained with PE, CD14 monocytes were stained with FITC, CD19 as a B cell marker was stained with allophycocyanin (APC), and CD3 as a T cell marker was stained with Brilliant Violet 421^{®} (BV421^{®}). The stained PBMCs were sorted into CD14 positive monocyte, CD19 positive B cell, and CD3 positive T cell (CD4 and CD8) fractions using BD FACS Aria^{®} Fusion cell sorter.

The CD14-positive monocyte fraction showed a mean fluorescence intensity of 1,622 in the cells cultured with PBS, whereas the mean fluorescence intensity was 42,287 in the cells cultured with rLVRN. The CD3-positive T cell fraction showed a mean fluorescence intensity of 372 in the cells cultured with PBS, whereas the mean fluorescence intensity was 3,678 in the cells cultured with rLVRN. These results indicate that laeverin induces ISG15 expression in any of the CD14-positive monocyte fraction, the CD19-positive B cell fraction, and the CD3-positive T cell fraction, and the induced ISG15 expression was remarkable in the CD14-positive monocyte fraction.

Cell immunostaining was conducted to identify further cells induced by rLVRN to express ISG15.

EDTA-treated blood 3 ml was processed with Ficoll to prepare PBMCs. The PBMCs were cultured in a Floating Cells Dish containing 2 ml of RPMI medium supplemented with rLVRN 1.5 µg/ml. After 24 hours of culture, the cells were collected and fixed by treatment with 4% paraformaldehyde for 10 minutes. The fixed cells were subjected to membrane permeabilization with PBS containing 0.1% Triton for 10 min. ISG15 was stained with PE. Mouse or rabbit antibodies to the following markers were used as primary antibodies and Alexa488-labeled anti-mouse or anti-rabbit antibodies were used as secondary antibodies: CD14 as a monocytic marker, CD19 as a B cell marker, CD4 as a T cell marker, and CD8 as a T-cell marker. In addition, cell nuclei were stained with Hoechst.

Cells were stained to observe the cell samples under a fluorescence microscope. This showed that laeverin induced IGS15 expression in all CD14-positive monocyte, CD19-positive cellular B cell, CD4-positive T cell, and CD8-positive T cell.

Examined were which types of cells took up rLVRNs to induce ISG15 expression and whether rLVRN induced ISG15 expression in a time-dependent manner. EDTA-treated blood 3 ml was processed with Ficoll to prepare PBMCs. The PBMCs were cultured for 24 hours in a Floating Cells Dish containing 2 ml of RPMI medium. After that, FITC-labeled rLVRN1.5 µg/ml was added to the medium and incubated for another 4 or 24 hours. The cultured cells were collected, and the PBMCs were sorted into monocyte and lymphocyte fractions using BD FACS Aria^{®} Fusion cell sorter.

The lymphocyte fraction showed that 0.7% of cells took up FITC-labeled rLVRN after 4 hours of rLVRN addition and 0.6% after 24 hours. The monocyte fraction showed that 25.4% of cells took up FITC-labeled rLVRN after 4 hours of rLVRN addition and 96.6% after 24 hours. These results indicate that monocytes, but not lymphocytes, take up laeverin and that the uptake of laeverin by monocytes is time-dependent.

Example 8 indicates that after uptake by monocytes, laeverin induces the expression of certain genes, such as ISG15, in both monocytes and lymphocytes.

### [EXAMPLE 9] Differentiation induction of mononuclear cells into dendritic cells by laeverin

Examined was how laeverin affected mononuclear cells that had taken up it.

EDTA-treated blood 3 ml was processed with Ficoll to prepare PBMCs. The PBMCs were cultured in a Floating Cells Dish containing 10 ml of RPMI medium. The cultured cells were collected, and the obtained cell suspension was mixed with CD 14 Micro Beads. The mixture was sorted into CD14-positive cells using Magnetic Activated Cell Sorting (MACS). A portion of the sorted CD14 positive cells was duplicated to culture wells, and rLVRN 1.5 µg/ml or PBS was added to each well. After 24 hours of culture, the cultured cells were observed under a microscope. RNA was extracted from the cultured cells and subjected to RT-qPCR to measure the expression levels of four genes: IFIT1, IFIT3, ISG15, and OAS2.

When CD14-positive cells derived from human peripheral blood mononuclear cells were cultured in the presence of rLVRN (lower panels of Figures 10a and 10b), morphological changes, such as cluster formation, were observed compared to those cultured in the presence of PBS (upper panels of Figures 10a and b). These micrographs suggest that monocytes differentiated into dendritic cells.

The table below shows the expression levels of the four genes in CD14-positive cells cultured in the presence of rLVRN relative to the expression levels of the genes in CD14-positive cells cultured in the presence of PBS. The table indicates that laeverin induces the genes to express in CD14-positive cells.

**[Table 4]**

| | IFIT1 | IFIT3 | ISG15 | OAS2 |
|---|---|---|---|---|
| Subject A | 2077 | 278 | 113 | 61 |
| Subject B | 7231 | 254 | 491 | 116 |
| Subject C | 37 | 13 | 31 | 7 |
| Subject D | 1448 | 145 | 613 | 135 |
| mean value | 2698 | 173 | 312 | 80 |
| standard deviation | 3140 | 121 | 284 | 58 |

The microscopic observations and RT-qPCR results suggest that Laeverin differentiates monocytes into dendritic cells, inducing the expression of the four genes in the differentiated dendritic cells. Subject C experienced a small effect of Laeverin inducing the four genes' expression. Subject C was undergoing fertility treatment.

Dendritic cells (DC) may be divided into four types according to their mode of presenting antigens: Dendritic cells (XCR1⁺ DC) of presenting foreign antigens in an MHC class I-dependent manner; Dendritic cells (CD11b⁺ DC) of presenting antigens in an MHC class II-dependent manner; Plasmacytoid dendritic cells (pDC) of producing large amounts of type I IFN during viral infection, and Dendritic cells differentiated from peripheral blood monocytes (moDC) during inflammation. The table below lists dendritic cell markers common in humans and mice.

**[Table 5]**

| XCR1⁺ DC | CD11b⁺ DC | pDC | moDC |
|---|---|---|---|
| MHC Class II | MHC Class II | MHC Class II | MHC Class II |
| CD11b(-) | CD11b(+) | CD123 | CD11c |
| CD11c | CD11c | CD4 | CD11b |
| FLT3 | FLT3 | CD14 | CX₃CR1 |
| XCR1 | CD11b | TLR7 | |
| CLEC9A | CX3CR1 | TLR9 | |
| CD14 | CD14 | | |
| CCR7 | CD4 | | |
| TLR3 | CCR7 | | |

Examined was which type of dendritic cells were differentiated by laeverin. CD14-positive cells derived from PBMCs were cultured in the presence of rLVRN, and RNA was extracted from the cultured CD14-positive cells. The RNA was subjected to RT-qPCR for specific genes (CD14, CD11c, CD123, CD80, and CD86) (FIG. 11a). PBMCs and THP -1 each cultured with rLVRN were also subjected to RT-qPCR in the same way (FIG.s 11b and 11c).

In CD14-positive cells, CD14 expression level decreased, CD123, CD80, and CD86 expression levels increased, and CD11c expression level was unchanged (FIG. 11a). Peripheral blood mononuclear cells (PBMCs) indicated a similar expression pattern to the CD14-positive cells for the above specific genes, but the changes in the expression level were more pronounced in the CD14-positive cells (FIG.s 11a and 11b). THP-1 indicated a different expression level pattern from PBMCs and CD14-positive cells for the specific genes (FIG.s 11a to 11c).

Example 9 demonstrates that laeverin can differentiate PBMCs-derived CD14-positive cells into dendritic cells, the dendritic cells characterized by decreased expression levels of CD14 (↓), increased expression levels of CD123 (↑), CD80 (↑), and CD86 (↑), and unchanged expression levels of CD11c (→) compared to CD14-positive cells. Focusing on CD11c and CD123, the differentiated dendritic cells induced by laeverin showed CD11c (+) CD 123 (+). As seen from Table 4, the differentiated dendritic cells induced by laeverin are considered distinct from the dendritic cell subsets, XCR1⁺ DCs, CD11b⁺ DCs, and moDCs since they are CD11c (+) CD123 (-). The differentiated dendritic cells induced by laeverin are considered distinct from the pDC subset since pDCs are CD11c (-) CD123 (+).

### [EXAMPLE 10] Dendritic cells differentiated from PBMCs induced by Laeverin

Cell markers are investigated for dendritic cells differentiated from human peripheral blood mononuclear cells (PBMCs) induced by laeverin.

EDTA-treated blood 3 ml was processed with Ficoll to prepare PBMCs. The PBMCs were cultured for 24 hours in a Floating Cells Dish containing 2 ml of RPMI medium. After that, rLVRN 1.5 µg/ml or PBS (as a control) was added to the medium. After an additional 24 hours of culture, the cultured cells were collected and stained with a BV421-labeled anti-CD14 antibody, a PE-labeled anti-HLA-DR antibody, and an APC-labeled anti-CD83 antibody. CD14 was used as a monocytic marker, HLA-DR as an antigen-presenting cell (APC) marker, and CD83 as a mature dendritic cell marker. The stained cultured cells were subjected to the BD FACS Aria^{®} Fusion cell sorter.

When adult female a-derived PBMCs were cultured in an rLVRN-supplemented medium, 19.3% of the cells in a fraction showed high expression of HLA-DR and little or no CD14 expression (Q1: HLA-DR(+)CD14(-)) (FIG. 12a). Within the Q1 fraction, 22.6% of cells highly expressed CD83 (CD83(+)) (FIG. 12b). When adult female a-derived PBMCs were cultured in a PBS-supplemented medium, 12.2% of the cells were found in the Q1 fraction, with 6.5% of cells highly expressing CD83. Similar tests, as described above, were carried out with adult male b-derived PBMCs. These results are summarized in the table below.

**[Table 6]**

| Sample | Supplemented component | Cell population in Q1 fraction [%] | CD83(+) cell population in Q1 fraction [%]. |
|---|---|---|---|
| Adult female a-derived PBMCs | PBS | 12.2 | 6.5 |
| | rLVRN | 19.3 | 26.6 |
| Adult male b-derived PBMCs | PBS | 15.1 | 6.8 |
| | rLVRN | 6.5 | 21.4 |

In adult female a-derived PBMCs, the HLA-DR(+)CD14(-) cell population (Q1 fraction) increased from 12.2% to 19.3% when the supplemented component PBS was replaced with rLVRN. For adult male b-derived PBMCs, the same cell population decreased from 15.1% to 6.5%. These results suggest that rLVRN may not necessarily lead to an increase in the HLA-DR(+)CD14(-) cell population.

In adult female a-derived PBMCs, the CD83(+) cell population in the Q1 fraction increased from 6.5% to 26.6% when the supplemented component PBS was replaced with rLVRN. For adult male b-derived PBMCs, the same cell population increased from 6.8% to 21.4%. These results suggest that laeverin may lead to induce PBMCs into dendritic cells expressing mature dendritic cell markers.

Additional cell markers are investigated for dendritic cells differentiated from PBMCs induced by laeverin.

PBMCs were cultured as described above, and cultured cells were subjected to the BD FACS Aria^{®} Fusion cell sorter. The cultured cells were stained with a FITC-labeled anti-CD14 antibody, a PE-labeled anti-HLA-DR antibody, an APC-labeled anti-CD11c antibody, and a BV421-labeled anti-CD123 antibody. CD14 was used as a monocytic marker, HLA-DR as an antigen-presenting cell (APC) marker, CD11c as a dendritic cell marker (primarily moDC), and CD123 as a dendritic cell marker (primarily pDC).

When adult female c-derived PBMCs were cultured in an rLVRN-supplemented medium, the Q1 fraction (HLA-DR(+)CD14(-)) contained 17.3% of the cells. Within the Q1 fraction, 50.7% of the cells highly expressed CD11c and CD123 (CD11c(+)CD123(+)). When adult female c-derived PBMCs were cultured in a PBS-supplemented medium, 15.2% of the cells were found in the Q1 fraction, with 3.2% of the cells highly expressing CD11c and CD123. These results are summarized in the table below.

**[Table 7]**

| Sample | Supplemented component | Cell population in Q1 fraction [%] | CD11c(+)CD123(+) cell population in Q1 fraction [%] |
|---|---|---|---|
| Adult female c-derived PBMCs | PBS | 15.2 | 3.2 |
| | rLVRN | 17.3 | 50.7 |

In adult women c-derived PBMCs, the HLA-DR(+)CD14(-) cell population (Q1 fraction) increased from 15.2% to 17.3% when the supplemented component PBS was replaced with rLVRN. Within the Q1 fraction, the CD11c(+)CD123(+) cell population increased markedly from 3.2% to 50.7%.

The FACS analysis showed that culturing monocytes in the presence of rLVRN increased the CD 123 (+) cell population in the CD11c(+) fraction in the overall monocyte fraction. Within the increased CD11c(+)CD123(+) cell population, around 90% of the cells exhibited CD14(+)HLA-DR(+). These results suggest that rLVRN may increase the CD123(+) cell population in the CD11c(+) fraction, although there may be some variation among samples.

Example 10 demonstrates that culturing PBMCs in the presence of laeverin results in an increase of CD83(+) dendritic cells in the CD14(-) cell population. Example 10 demonstrates that CD11c(+) CD123(+) dendritic cells are increased in monocyte fractions, whether CD14-positive or negative cells.

Focusing on CD123 and CD11c, the differentiated dendritic cells from PBMCs induced by culturing them in the presence of laeverin were CD123(+)CD11c(+). As seen from Table 4, the differentiated dendritic cells induced by laeverin are considered distinct from the dendritic cell subsets, XCR1⁺ DCs, CD11b⁺ DCs, and moDCs since they are CD11c(+)CD123(-). The differentiated dendritic cells induced by laeverin are considered distinct from the pDC subset since pDCs are CD11c(-)CD123(+). The results are the same as in Example 9.

### [EXAMPLE 11] Expression level of IDO1 in differentiation-induced dendritic cells

PBMCs from subjects were cultured in the presence of rLVRN. The expression level of indoleamine 2,3-dioxygenase-1 (IDO1) in the cultured cells was analyzed using RT-qPCR. CD14-positive cells derived from PBMCs were cultured in the presence of rLVRN, and the expression level of IDO1 in the cultured cells was analyzed in the same manner as above (n=2). The expression level of IDO1 in PBMCs cultured in a medium supplemented with PBS (as a negative control) instead of rLVRN was also analyzed. The relative expression levels of IDO1 in cultured cells under each culture condition relative to the negative control were calculated (Table 7).

**[Table 8]**

| | PBMCs + rLVRN | (1st Trial) CD14-positive cells + rLVRN | (2nd Trial) CD14-positive cells + rLVRN |
|---|---|---|---|
| Subject A | | 1314 | 671 |
| Subject B | | 31652 | 1168 |
| Subject C | 26 | 380 | 170 |
| Subject D | | 221 | 53 |
| Subject E | 101 | | |
| Subject F | 5.5 | | |
| mean value | 44 | 8392 | 516 |
| standard deviation | 50 | 15514 | 511 |

When PBMCs were cultured in the presence of laeverin, a marked increase in the expression level of IDO1 was observed; when CD14-positive cells were cultured in the presence of laeverin, a marked increase in the expression level of IDO1 was observed.

Examples 9 and 11 suggest that CD14-positive cells taken up laeverin can differentiate into dendritic cells with high expression levels of IDO1.

### [Example 12] Effect of anti-laeverin antibody on cancer cells expressing laeverin

An anti-laeverin antibody was used for cancer cells expressing laeverin. The use of the monoclonal anti-laeverin antibody led to an observation of intracellular uptake of laeverin on the cell surface. Melanoma A375 cells were cultured by a hanging drop method (1,000 cells per culture medium drop of 20 µL) for one day. The cultured A375 cells formed cell aggregates, in which A375 cells expressing laeverin were observed (FIG. 13a). Melanoma A375 cells were cultured in the presence of pHrodo-conjugated anti-LVRN antibody by the hanging drop method (10 cells per culture medium drop of 20 µL) for one day. The cultured A375 cells formed 2 to 3 cell aggregates, in which A375 cells expressing laeverin were observed. It was observed that the A375 cells expressing laeverin took in the pHrodo-conjugated anti-LVRN antibody (FIG. 13b). The intracellular uptake of laeverin with anti-laeverin antibodies on the cell membrane was observed for all three antibodies (including the 5-23 antibody) that were utilized in this study.

The reduction of laeverin on the cell surface suggests that monocytes may not be adequately induced to differentiate into dendritic cells expressing IDO1, thereby exposing the cancer cells to the immune system attack. These findings suggest the anti-laeverin antibody could effectively treat recurrent and/or metastatic cancers involving cancer cells expressing laeverin.

The differences in gene expression patterns between a Swan71 cell transformant that overexpresses laeverin (Swan71_LVRN) and the transformant used with 5-23 antibodies were analyzed using microarray. The use of 5-23 antibodies resulted in the reduced expression of four specific genes and the IDO1 gene in Swan71-LVRN. The decrease in IDO1 gene expression in cancer cells due to anti-laeverin antibodies suggests that the cancer cells do not effectively evade immune system attacks, making them vulnerable to the immune system. The finding suggests that anti-laeverin antibodies could effectively treat recurrent and/or metastatic cancers involving cancer cells expressing laeverin.

The ovarian cancer cell line SKOV3 was cultured in suspension to form spheroids (FIG. 14a, upper panel). A cytotoxic agent, monomethyl auristatin E (MMAE), was conjugated to a monoclonal anti-laeverin antibody, 5-23 antibody, to prepare MMAE-conjugated 5-23 antibody. The cancer cell spheroids were cultured in the presence of the MMAE-conjugated 5-23 antibody for 7 days. After that, the cancer cell spheroids were stained with propidium iodide (PI). PI-stained cells (i.e., cell death) were observed (FIG. 14a, middle panel). The result suggests that anti-laeverin antibodies containing cytotoxic agents can effectively treat recurrent and/or metastatic cancers in which the presence of cancer cells expressing laeverin has been assessed.

The breast cancer cell line SKBR3 and melanoma SK-MEL28 were cultured in the same manner as SKOV3 to form cancer cell spheroids and cultured in the presence of MMAE-conjugated 5-23 antibodies. As a result, PI-stained cells (i.e., cell death) were observed in both cancer cell spheroids. These results suggest that treating recurrent and/or metastatic cancers with anti-laeverin antibodies conjugated with cytotoxic agents is effective in various cancer types.

The middle panel of Figure 14b shows the presence of PI-stained cells (i.e., cell death) in ovarian cancer cell spheroids cultured in the presence of MMAE-nonconjugated 5-23 antibodies alone. The result suggests that the anti-laeverin antibody itself (without conjugation to cytotoxic agents) can treat recurrent and/or metastatic cancers. No cell death-inducing effects of anti-laeverin antibodies themselves were observed in the breast cancer cell line SKBR3 and melanoma SK-MEL28. These results suggest that anti-laeverin antibodies themselves can induce cell death in cancer cells expressing laeverin depending on the type of cancer.

### [EXAMPLE 13] Cancer cells expressing laeverin involved in lymph node metastasis

Tissue around the lymph node of an ovarian cancer patient with metastatic cancer was collected and immuno-stained. Figure 15 shows immuno-stained images of a lymph node near an afferent lymphatic. Figure 16 shows immuno-stained images of the lymph node near an efferent lymphatic. Figure 15b shows the presence of a floating cancer cell aggregate in the afferent lymphatic. Figure 15c shows that cancer cells constituting the cell aggregate express laeverin. Figures 15d and 15e show the presence of cancer cells with laeverin expression at a low level around the tumor site within the metastasized lymph node. Figures 16a and 16b show the presence of cancer cell aggregates that detached from the tumor site within the metastasized lymph node and are present within the efferent lymphatic. Figures 16c to 16e show that the cancer cells constituting the cancer cell aggregates within the efferent lymphatic express laeverin. Figure 16c shows that cancer cells in metastasis sites within the lymph node do not express laeverin.

Example 13 demonstrates that cancer cells expressing laeverin that detached from the primary ovarian cancer tumor site can reach lymph nodes through afferent lymphatic, that cancer cells expressing laeverin that reach lymph nodes can proliferate within the lymph nodes to form metastatic tumor tissue (including cancer cells with low-level expression or no expression of laeverin), and that cancer cells expressing laeverin that detached from the metastatic tumor tissue can metastasize further through efferent lymphatic. Example 13 also demonstrates that cancer cells involved in lymph node metastasis strongly express laeverin under conditions that are harsh on cell survival, such as floating in lymph vessels, and become little or no expression of laeverin under conditions that allow them to adhere and survive such as in metastatic tumors.

Immune cells gather in lymphatic vessels and lymph nodes, and the immune activity is high. The Examples of this specification demonstrate a possibility that cancer cells expressing laeverin can evade immune system attacks due to the expression of IDO1 in the cancer cells themselves or evade further immune system attacks by inducing monocytes in contact with laeverin to differentiate into IDO1-expressing dendritic cells. These findings suggest that cancer cells that express or are capable of expressing laeverin can play an essential role in cancer metastasis and/or recurrence, including lymph node metastasis, especially in systemic metastasis.

### [EXAMPLE 14] Cancer cells surviving inside primary tumor tissue surrounded by necrotic cells

Primary tumor tissue from an ovarian cancer patient with metastatic cancer contained necrosis in extensive areas (FIG. 17a). Remaining tumor was found in the area surrounded by the necrosis areas inside the primary tumor tissue. The remaining tumor expressed laeverin (FIG. 17b). In the necrosis areas, no expression of laeverin was observed (FIG. 17c). In the region containing surviving cancer cells outside of the necrosis areas, no expression of laeverin was observed (FIG. 17d and FIG. 17e).

Blood vessels were observed in the remaining tumor area surrounded by the necrotic cells inside the primary tumor tissue, but no blood vessels were observed in the necrosis areas.

Example 14 demonstrates that cancer cells expressing laeverin can survive even under severe conditions for cell survival, such as conditions allowing cancer cell necrosis, or that when such severe conditions are encountered, cancer cells capable of expressing laeverin express laeverin and maintain survival. The findings of Examples 13 and 14 suggest that cancer cells capable of expressing laeverin can express laeverin and maintain survival when the situation becomes severe for cell survival. Example 14 demonstrates that blood vessels were present around the cancer cells expressing laeverin. The finding suggests that it is possible to treat cancer cells expressing laeverin, which can play an essential role in cancer recurrence and/or metastasis, with anticancer agents injected into the blood.

### [EXAMPLE 15] Laeverin in chemotherapy-resistant cancer cells

### (Specimen E)

A patient with stage IIIC ovarian cancer underwent TC therapy as neoadjuvant therapy (NAC) followed by tumor reduction surgery (IDS) including abdominal total hysterectomy (ATH), bilateral adnexectomy (BSO), and oophorectomy (OMT). After the IDS, the patients underwent TC therapy. The ovary removed during the IDS was labeled as Specimen E. Tissue sections from the excised ovary were prepared, stained with HE, and subjected to immunostaining with anti-laeverin antibodies.

In ovarian cancer after the TC therapy, necrotic tumor tissue and small remaining tumor tissue were found (FIG. 18a). The majority of the remaining tumor tissue was surrounded by necrotic tumor tissue. The remaining tumor tissue consisted of cancer cells expressing laeverin (FIG. 18b).

### (Specimen F)

A patient with stage IIIC ovarian cancer underwent the combination therapy of docetaxel and carboplatin (DC therapy) as NAC, followed by IDS including ATH, BSO, OMT, and low anterior rectal resection. After the IDS, the patient underwent DC therapy. One year after the DC therapy, the patient had a recurrent peritoneal dissemination. The ovary removed during the IDS was labeled as Specimen F. Tissue sections from the excised ovary were prepared, stained with HE, and subjected to immunostaining with anti-laeverin antibodies.

In ovarian cancer after DC therapy, several cancer cells were found separately within the necrotic major tissues (FIG. 18c). The cancer cells were surrounded by necrotic tumor tissue and expressed Laeverin (FIG. 18d).

Example 15 demonstrates that cancer cells resistant to chemotherapy express laeverin. The finding suggests that cancer cells expressing or capable of expressing laeverin are resistant to cancer therapies, including chemotherapy, and may play an essential role in cancer recurrence and/or metastasis, especially local recurrence.

### [EXAMPLE 16] Effect of anti-laeverin antibody in mice transplanted with cancer cells

The ovarian cancer cell line A2780 was transformed to produce A2780 LVRN. As a control, the A2780 was transfected with an empty vector CAG to produce A2780 CAG (control).

Female Balb/c nu/nu mice (6-8 weeks old) were injected with LA2780 LVRN at two sites on the right side and LA2780 CAG at two sites on the left side, both at 5 × 10⁶ cells/100 µ per site, to generate mice transplanted with cancer cells. Ten mice with transplanted cancer cells were obtained.

Two and eight days after the injection of A2780, five mice with transplanted cancer cells were injected with MMAE-conjugated 5-23 antibodies (10 mg/kg) through the tail, and their tumor sizes were measured. The remaining five mice with transplanted cancer cells were injected with PBS through the tail at two and eight days after the injection of A2780, and their tumor sizes were measured., Fifteen days after the injection of A2780, the tumor sizes of the ten mice with transplanted cancer cells were measured.

Figure 19 demonstrates that the tumor size reached approximately 300 mm³ on day 15 after the injection of A2780 LVRN when A2780 LVRN transplanted mice were treated with PBS. When A2780 LVRN transplanted mice were treated with MMAE-conjugated 5-23 antibody, no tumors were visually observed on day 15 after the injection of A2780 LVRN.

Example 16 demonstrates that tumors formed by cancer cells expressing laeverin in vivo are treated with anti-laeverin antibodies containing chemotherapeutic agents.

### [Example 17] Cancer expressing laeverin

Three patients suffered from placental-site trophoblast tumors (PSTT) underwent a surgery to give PSTT tissue, respectively. Frozen sections were prepared from the PSTT tissues. The frozen sections were subjected to immunostaining with 5-23 antibody. The immunostaining revealed that the overall tumor tissue expressed laeverin.

Example 17 suggests that a pharmaceutical composition containing an anti-laeverin antibody can treat tumors expressing laeverin (e.g., PSTT).

## Claims

1. A pharmaceutical composition for treatment of cancer, comprising a substance binding to laeverin.

2. The pharmaceutical composition according to claim 1, wherein the substance binding to laeverin comprises an anti-laeverin antibody.

3. The pharmaceutical composition according to item 2, wherein the anti-laeverin antibody comprises
(A)
a heavy chain variable region comprising
a CDR-H1 comprising an amino acid sequence: GYSFTDYI (SEQ ID NO. 1);
a CDR-H2 comprising an amino acid sequence: INPYHAGI (SEQ ID NO. 2); and
a CDR-H3 comprising an amino acid sequence: ARGSNYVYYYAMD (SEQ ID NO. 3), and/or
a light chain variable region comprising
a CDR-L1 comprising an amino acid sequence: SSVSY (SEQ ID NO. 4);
a CDR-L2 comprising an amino acid sequence: ATS; and
a CDR-L3 comprising an amino acid sequence: QQWSSNPPT (SEQ ID NO. 5),
(B)
a heavy chain variable region comprising
a CDR-H1 comprising an amino acid sequence: GYTFTSYW (SEQ ID NO. 11);
a CDR-H2 comprising an amino acid sequence: IDPYDSET (SEQ ID NO. 12); and
a CDR-H3 comprising an amino acid sequence: ARDYGSRYYAMD (SEQ ID NO. 13), and/or
a light chain variable region comprising
a CDR-L1 comprising an amino acid sequence: ENVVTY (SEQ ID NO. 14);
a CDR-L2 comprising an amino acid sequence: GAS; and
a CDR-L3 comprising an amino acid sequence: GQGYSYP (SEQ ID NO. 15),
(C)
a heavy chain variable region comprising
a CDR-H1 comprising an amino acid sequence: GYTFTSYW (SEQ ID NO. 11);
a CDR-H2 comprising an amino acid sequence: IDPYDSET (SEQ ID NO. 12); and
a CDR-H3 comprising an amino acid sequence: ARDYGSRYYAMD (SEQ ID NO. 13), and/or
a light chain variable region comprising
a CDR-L1 comprising an amino acid sequence: STISY (SEQ ID NO. 16);
a CDR-L2 comprising an amino acid sequence: DTS; and
a CDR-L3 comprising an amino acid sequence: QQWSSNPP (SEQ ID NO. 17), or
(D)
a heavy chain variable region comprising
a CDR-H1 comprising an amino acid sequence: GYTFTDYY (SEQ ID NO. 18); and
a CDR-H2 comprising an amino acid sequence: IYPRSGHS (SEQ ID NO. 19), and/or
a light chain variable region comprising
a CDR-L1 comprising an amino acid sequence: QSLLYSNIQKNY (SEQ ID NO. 20);
a CDR-L2 comprising an amino acid sequence: WAS; and
a CDR-L3 comprising an amino acid sequence: QQYYSYP (SEQ ID NO. 21).

4. The pharmaceutical composition according to claim 2 or 3, wherein the anti-laeverin antibody possesses an ADCC activity or a CDC activity.

5. The pharmaceutical composition according to any one of claims 1 to 4, wherein the substance binding to laeverin comprises a cytotoxic agent.

6. The pharmaceutical composition according to claim 5, wherein the cytotoxic agent comprises at least one selected from the group consisting of a radioisotope, a chemotherapeutic agent, a toxin, and an enzyme.

7. The pharmaceutical composition according to any one of claims 1 to 6, wherein the cancer is recurrent cancer, metastatic cancer or resistant cancer, or choriocarcinoma, placental trophoblast tumor, ovarian cancer, fallopian tube cancer, uterine cancer, cervical cancer, breast cancer, mammary gland cancer, glioblastoma, colon cancer, prostate cancer, or leukemia.

8. A method for assessing cancer recurrence, metastasis, or resistance, comprising:
detecting a cancer cell expressing laeverin in a biological sample derived from a subject in need thereof by using a substance binding to laeverin; and
assessing recurrence, metastasis, or resistance of cancer, based on the detection result.

9. A method for determining a substance impairing a cancer cell expressing laeverin, comprising:
culturing a cancer cell expressing laeverin under a condition that allows contact with a test substance, and
determining the test substance as a substance impairing a cancer cell expressing laeverin, based on the culture result.

10. A method for determining a factor influencing cancer recurrence, metastasis, or resistance, comprising:
comparing a cancer cell expressing laeverin with a cancer cell that does not express laeverin but has the same origin as the cancer cell expressing laeverin to determine a difference in genetic or proteinaceous factor.

11. A use of laeverin as a biomarker for a cancer stem cell.
